# EUROPEAN PATENT SPECIFICATION

(11) **EP 2 970 463 B1**
(45) Date of publication and mention of the grant of the patent: **29.11.2017**
(21) Application number: 14709335.5
(22) Date of filing: 12.03.2014
(51) Int. Cl.: C07K 16/28, C07K 16/46, C07K 14/47

(54) **METHODS FOR TREATING AND/OR LIMITING DEVELOPMENT OF DIABETES**
VERFAHREN ZUR BEHANDLUNG UND/ODER BEGRENZUNG DER ENTWICKLUNG VON DIABETES
PROCÉDÉS POUR TRAITER LE DIABÈTE ET/OU LIMITER SON ÉVOLUTION

(30) Priority: 13.03.2013 US 201361779508 P
(43) Date of publication of application: 20.01.2016
(73) Proprietor: BioCrine AB, 169 03 Solna (SE)
(72) Inventor: BERGGREN, Per-Olof, S-16955 Solna (SE); JUNTTI-BERGGREN, Lisa, S-16955 Solna (SE)
(74) Representative: Zwicker, Jörk
(86) International application number: PCT/EP2014/054862
(87) International publication number: WO 2014/140113

(56) References cited:
- WO-A1-2007/097751
- WO-A1-2010/083615
- US-A1- 2003 032 594
- US-A1- 2004 224 304
- US-A1- 2012 328 630
- J.R. WEAVER ET AL: "Regulation of NOX-1 expression in beta cells: A positive feedback loop involving the Src-kinase signaling pathway", MOLECULAR AND CELLULAR ENDOCRINOLOGY, vol. 369, no. 1-2, 11 February 2013 (2013-02-11), pages 35-41, XP55120047, ISSN: 0303-7207, DOI: 10.1016/j.mce.2013.01.011
- S. UEBERBERG ET AL: "Protection from diabetes development by single-chain antibody-mediated delivery of a NF- B inhibitor specifically to -cells in vivo", AJP: ENDOCRINOLOGY AND METABOLISM, vol. 301, no. 1, 1 July 2011 (2011-07-01), pages E83-E90, XP55120265, ISSN: 0193-1849, DOI: 10.1152/ajpendo.00603.2010
- R. HOLMBERG ET AL: "Lowering apolipoprotein CIII delays onset of type 1 diabetes", PROCEEDINGS OF THE NATIONAL ACADEMY OF SCIENCES, vol. 108, no. 26, 13 June 2011 (2011-06-13), pages 10685-10689, XP55102683, ISSN: 0027-8424, DOI: 10.1073/pnas.1019553108
- YUE SHI ET AL: "Apolipoprotein CIII hyperactivates [beta] cell CaV1 channels through SR-BI/[beta]1 integrin-dependent coactivation of PKA and Src", CELLULAR AND MOLECULAR LIFE SCIENCES, 15 August 2013 (2013-08-15), XP55102669, ISSN: 1420-682X, DOI: 10.1007/s00018-013-1442-x

## Description

### CROSS REFERENCE

This application claims priority to U.S. Provisional Patent Application Serial No. 61/779508 filed March 13, 2013.

### BACKGROUND OF THE INVENTION

The International Diabetes Federation (IDF) has estimated that the number of people with diabetes in 2011 was 366 million and this number is expected to grow to 550 million by 2030. The majority of patients are suffering from type-2 diabetes mellitus (T2DM). There is a link between obesity, insulin resistance and the development of T2DM, but the precise underlying mechanisms remain unknown and it becomes paramount to identify potential therapeutic targets that can ultimately address this crucial problem.

Classical target tissues subjective to insulin resistance in T2DM are muscle, liver and fat. These peripheral tissues maintain glucose homeostasis by effectively responding to insulin and failure of insulin to activate its receptors and the downstream signaling cascades result in defect glucose handling. In addition, insulin can stimulate its receptors on the pancreatic β-cell and thereby directly contribute to a positive feedback loop for insulin biosynthesis and secretion.

Apolipoprotein CIII (apoCIII) is a lipoprotein synthesized mainly in the liver, and to a lesser extent in the small intestine. ApoCIII resides on apoB lipoproteins and high density lipoproteins (HDL) and regulates their metabolism by inhibiting lipoprotein lipase (LPL). The expression of apoCIII is increased by insulin deficiency and insulin resistance and it has also been shown that hyperglycemia induces apoCIII transcription. On top of high levels of circulating triglycerides, mice overexpressing apoCIII are more susceptible to high-fat diet induced diabetes. In humans, elevated levels of circulating apoCIII are associated with insulin resistance. Furthermore, patients with type-1 diabetes mellitus (T1DM) were found to have elevated levels of serum apoCIII. This resulted in a higher activity of the voltage-gated Ca²⁺- channels, increased cytoplasmic free Ca²⁺ concentration ([Ca²⁺]ᵢ) and apoptosis, an effect that was blocked by lowering apoCIII.

### SUMMARY OF THE INVENTION

The invention as disclosed herein provides a composition comprising a compound of formula A-B, wherein A is a pancreatic β cell targeting moiety, and B is an inhibitor of expression and/or activity of Apolipoprotein CIII (apoCIII) and/or β1 integrin for use in treating or limiting development of diabetes in a subject.

In another aspect, a composition comprising a compound of formula A-B is provided, wherein A is a pancreatic β cell targeting moiety, and B is an inhibitor of Apolipoprotein CIII (apoCIII) or β1 integrin. In an additional aspect, the composition comprises a compound of formula A-B-C, wherein C is a compound that permits cell entry of the composition.

In various embodiments, the apoCIII inhibitor is selected from the group consisting of anti-apoCIII antibody, anti-apoCIII aptamer, apoCIII small interfering RNA, apoCIII small internally segmented interfering RNA, apoCIII short hairpin RNA, apoCIII microRNA, and apoCIII antisense oligonucleotides.

In various embodiments, the β1 integrin inhibitor is selected from the group consisting of anti-β1 integrin antibody, antiβ1 integrin aptamer, β1 integrin small interfering RNA, β1 integrin small internally segmented interfering RNA, β1 integrin short hairpin RNA, β1 integrin microRNA, and β1 integrin antisense oligonucleotides.

In various embodiments, the pancreatic β cell specific targeting moiety comprises a moiety that that selectively binds a protein selected from the group consisting of DiGeorge syndrome critical region gene 2 (DGCR2), golgi brefeldin A resistant guanine nucleotide exchange factor 1 (GBF1), orphan G protein-coupled receptor GPR44(GPR44), SerpinB10 (PI-10), FXYD domain containing ion transport regulator 2 (FXYD2), Tetraspanin-7 (TSPAN7), gap junction protein, delta 2, 36kDa (GJD2), solute carrier family 18 (vesicular monoamine), member 2 (SLC18A2), prokineticin receptor 1 (PROKR1), glutamate receptor, metabotropic 5 (GRM5), neuropeptide Y receptor Y2 (NPY2R), glucagon-like peptide 1 receptor (GLP1R), and transmembrane protein 27 (TMEM27). In further embodiments, the pancreatic β cell specific targeting moiety comprises a moiety selected from the group consisting of glucagon-like peptide-1 (GLP-1), glucagon-like peptide-2 (GLP-2), peptide YY (PYY), neuropeptide Y (NPY), pancreatic peptide (PP), exendin-4, naphthylalanine and naphthylalanine derivatives.

Specific embodiments of the invention will become evident from the following more detailed description of certain embodiments and the claims.

### BRIEF DESCRIPTION OF THE DRAWINGS

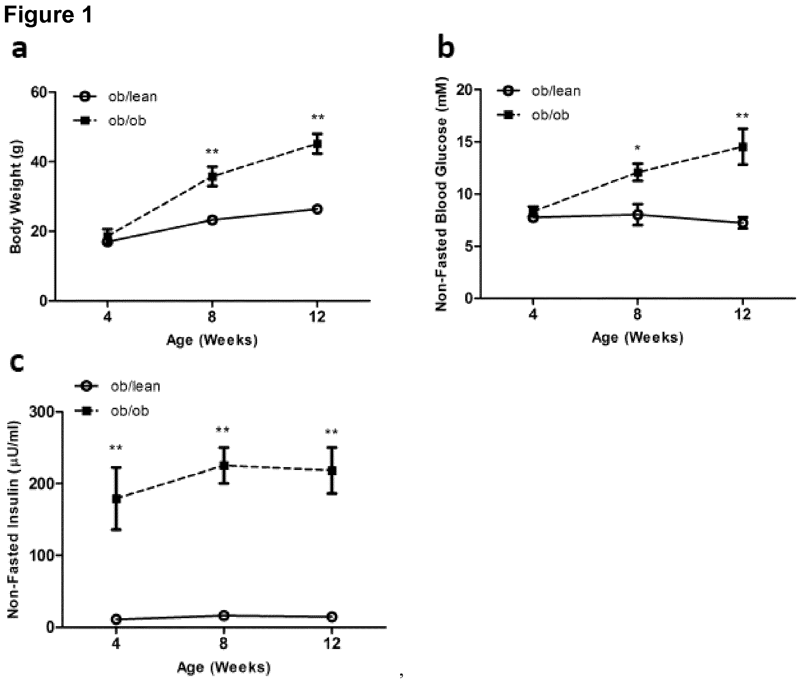

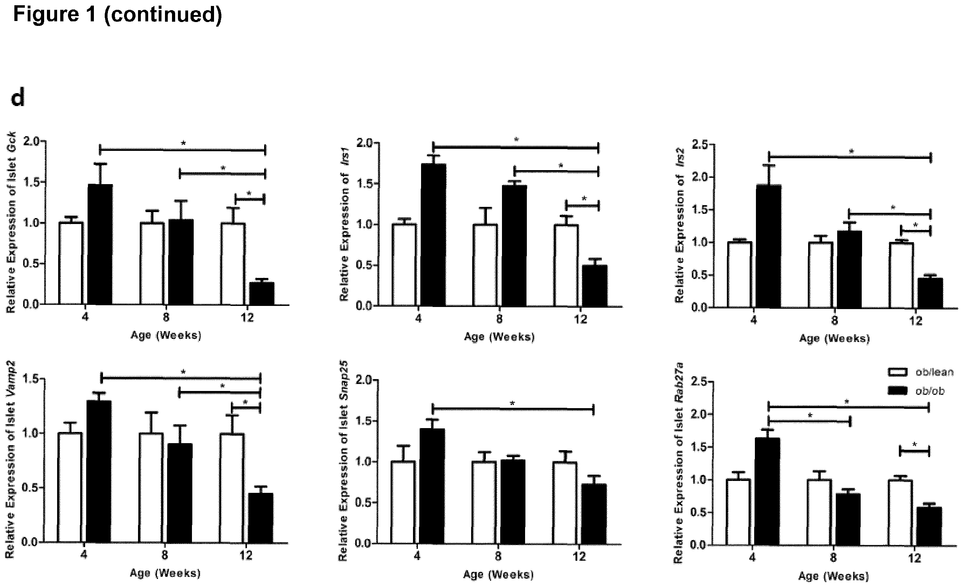

**Figure 1** shows exacerbated hyperglycemia and reduced insulin signaling in ob/ob islets across time. **(a)** Average body weight, **(b)** non-fasted blood glucose levels and **(c)** non-fasted plasma insulin levels measured in both ob/ob and age-matched ob/lean mice at 4-, 8- and 12-weeks of age (n=8-11). **(d)** Pancreatic islet gene expression changes between obese and lean controls at 4-, 8- and 12-weeks of age. mRNA levels were determined by real-time qPCR, normalized to β-actin. Expression levels of ob/ob were compared to those of ob/lean, which were set at 1.0. There was no significant change in ob/lean gene expression over time (n=4-9). Data presented as mean ± standard error of the mean (SEM). *P<0.05, ** P<0.01 by student's t-test or ANOVA (Tukey's post-hoc analysis).

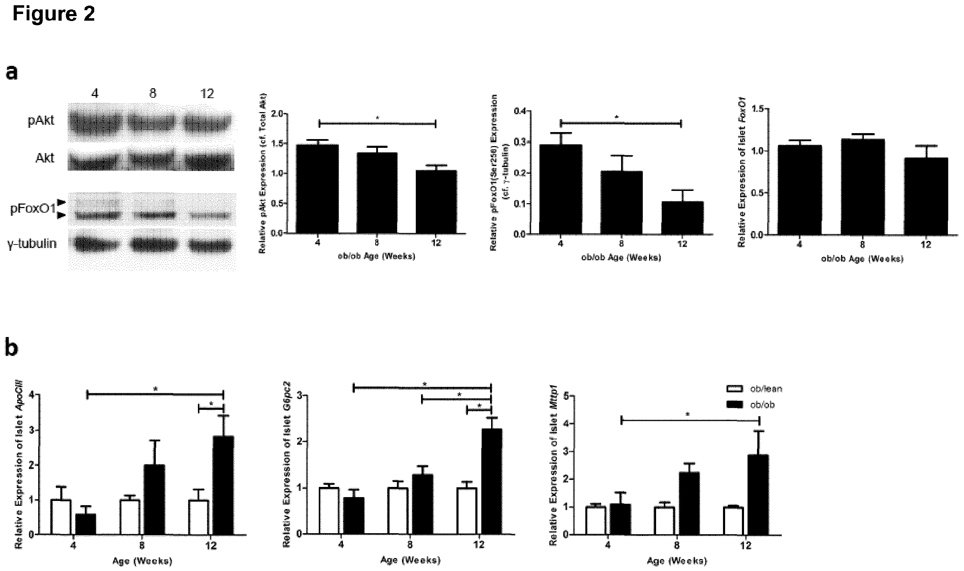

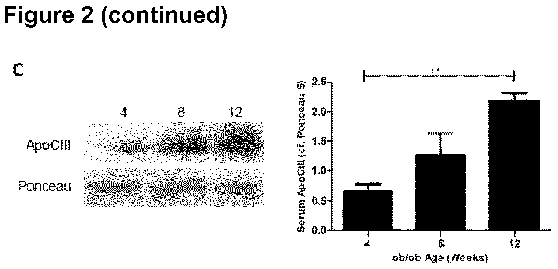

**Figure 2** shows changes in insulin signaling cascade in ob/ob islets across time. **(a)** Western blot analysis of ob/ob islet lysates prepared from animals at 4-, 8- and 12-weeks of age, probed with antibodies specific for phosphorylated -Akt, total Akt, phosphorylated-FoxO1 and γ-tubulin. Bands were quantitated by densitometry. Total FoxO1 immunoblots had poor signal to noise ratios and thus mRNA levels of FoxO1 were measured instead (n=3-4). **(b)** Pancreatic islet gene expression changes between obese and lean controls at 4-, 8- and 12-weeks of age. mRNA levels were determined by real-time qPCR, normalized to β-actin (n=4-8). **(c)** Western blot of serum apoCIII with Ponceau S stain as loading control along with the respective densitometry quantitation (n=3). Data presented as mean ± SEM. *P<0.05, ** P<0.01 by ANOVA (Tukey's post-hoc analysis).

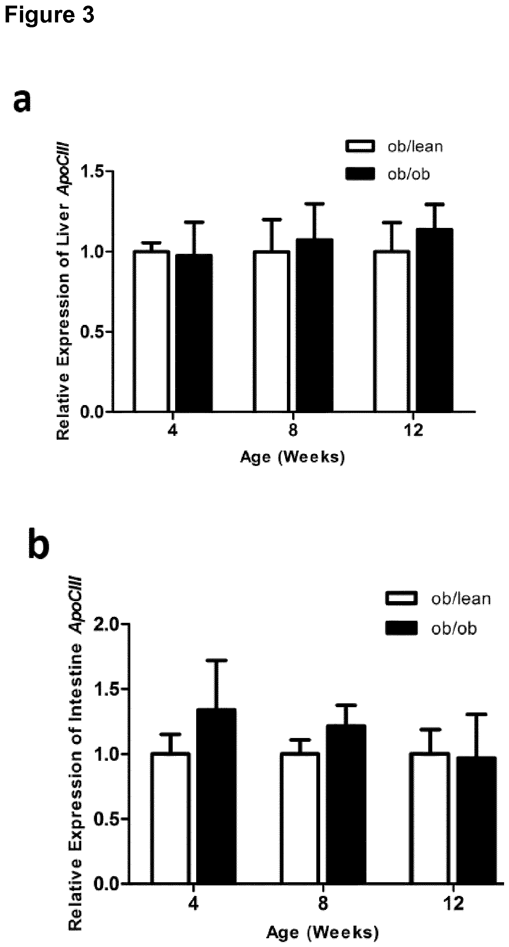

**Figure 3** shows the relative expression of *apoCIII* from tissues known to express apoCIII. **(a)** Liver expression of *apoCIII* in both ob/lean and ob/ob at age 4, 8 and 12 weeks are compared and contrasted (n = 5-7). **(b)** Small intestine expression of *apoCIII* in both ob/lean and ob/ob at age 4, 8 and 12 weeks are compared and contrasted (n = 5-6). mRNA levels were determined by real-time qPCR, normalized to β-action. Intron-spanning (exon 3 and 4) real-time qPCR primers used were exactly the same as that used for determining *apoCIII* expression from pancreatic islet lysates. Data presented as mean ± SEM.

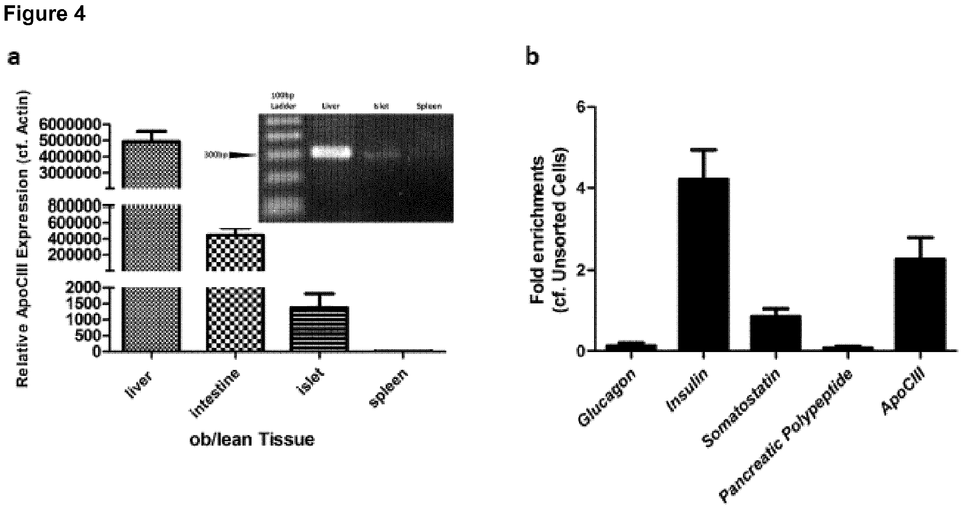

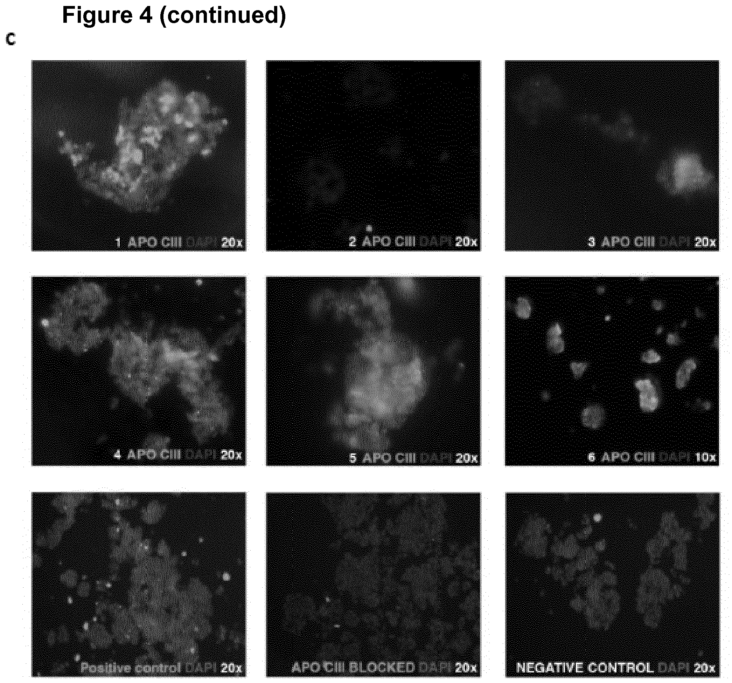

**Figure 4** shows expression of apoCIII in rodent and human pancreatic islets. **(a)** Tissue-specific expression of apoCIII in ob/lean mice. Specific intron-spanning primers were used to determine apoCIII expression by real-time qPCR. Insert shows representative amplification of the entire coding region of apoCIII in the different tissues (n=4-6). **(b)** ApoCIII expression in sorted rat primary β-cells. Single cells from dissociated rat islets were sorted according to their distinct auto-fluorescence. Cells were subsequently lysed for expression analysis by real-time qPCR. The enrichment of different islet hormones in the sorted cells shows purity of the sorting method (n=4). **(c)** ApoCIII localization in isolated human islets using an antibody that recognizes the human apoCIII epitope. As controls, apoCIII antibodies were preincubated/blocked with recombinant apoCIII prior to use. In the negative control, only the secondary antibody was added (n=3-6). Data presented as mean ± SEM.

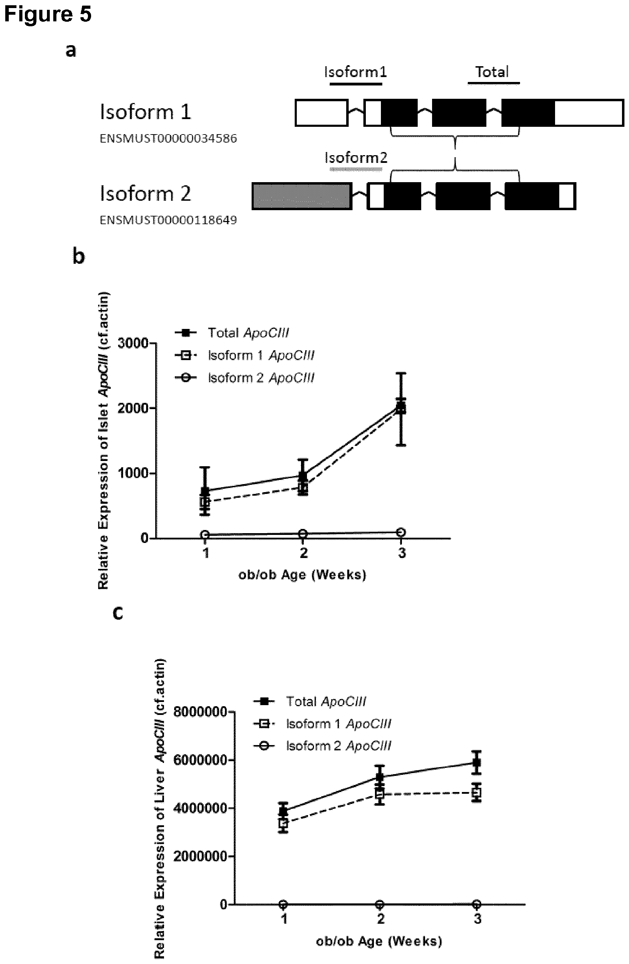

**Figure 5** shows the relative expression of the different *apoCIII* isoforms. **(a)** Illustration showing the difference between the two isoforms of *apoCIII* as determined by ENSEMBL (transcript ID as shown). Both isoforms have the similar number of exons and the exact same coding sequence (black filled bars). They only differ in the 5' untranslated region (UTR) corresponding to exon 1 with isoform 2 having a longer 5'UTR (grey filled bar) compared to isoform 1. Isoform 2 also has a shorter 3'UTR in exon 4. Therefore, different intron spanning real-time qPCR primers were employed to determine the relative levels of both isoform 1 and isoform 2 based on the different sequences of exon 1. The different primers shown were tested and shown to have similar priming efficiencies. **(b)** Relative expression the 2 different *apoCIII* isoforms in pancreatic islet lysates (n = 3). **(c)** Relative expression of the 2 different *apoCIII* isoforms in liver lysates (n = 3). Data presented as mean ± SEM.

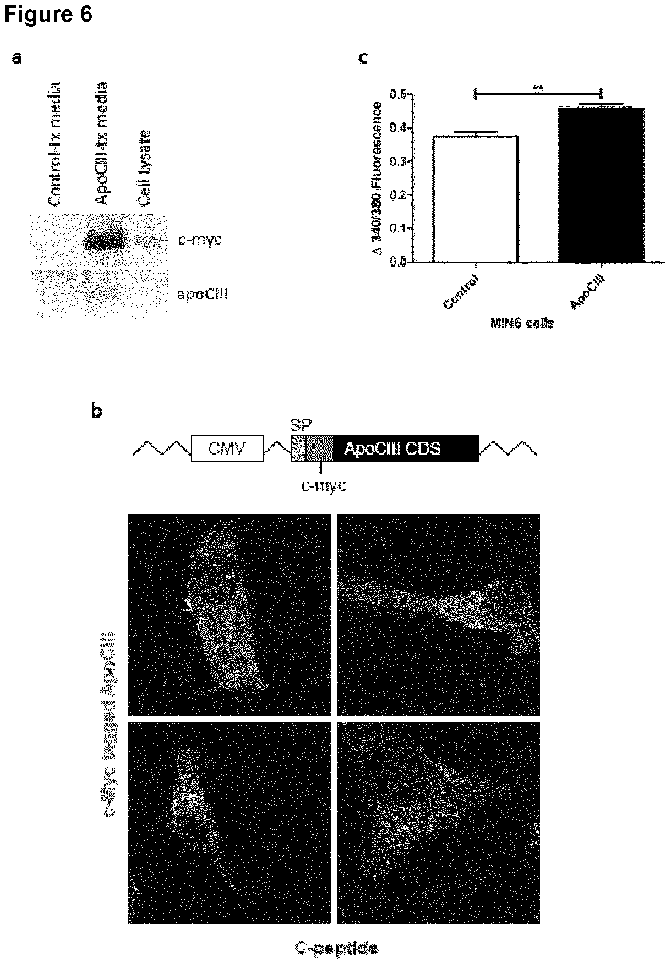

**Figure 6** shows overexpression of mouse Myc-apoCIII in Min6 cells and its implications for Ca²⁺ influx. **(a)** Both media and cells were collected 24 hours post transfection. C-Myc antibody was used for immunoprecipitation and the resulting bound protein was probed with both c-myc and apoCIII antibody. ApoCIII is detected in the apoCIII transfected cell culture media, but neither detected in the lysate nor in the control transfected cell culture media. **(b)** Mouse apoCIII coding sequence was tagged with a c-myc sequence right after the signal peptide and expressed in Min6 cells. As a control, a ds-tomato construct under the same promoter was used. Representative maximum intensity projection images of c-Myc and C-peptide staining in transfected Min6 cells. **(c)** Both Myc-apoCIII and control transfected Min6 cells were loaded with Fura-2 to determine [Ca²⁺]ᵢ upon membrane depolarization using 25 mM KCl (n=4). Data presented as mean ± SEM. ** P<0.01 by student's t-test.

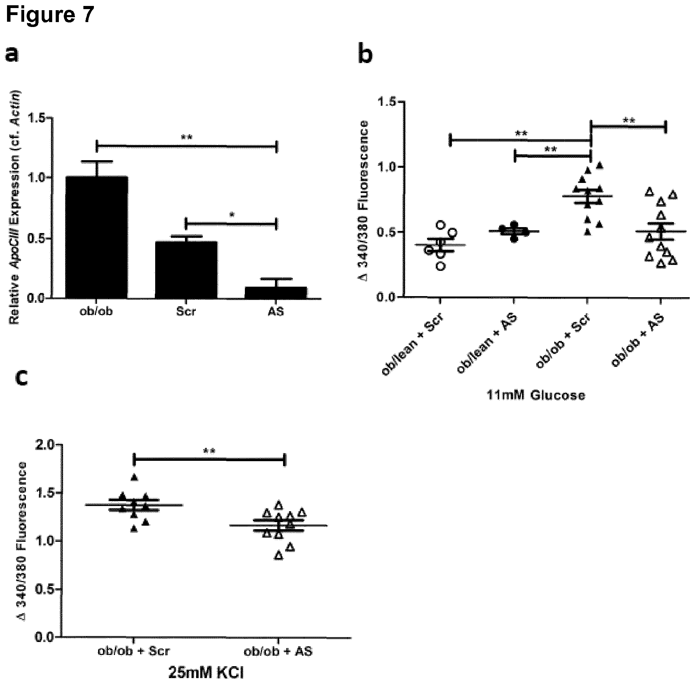

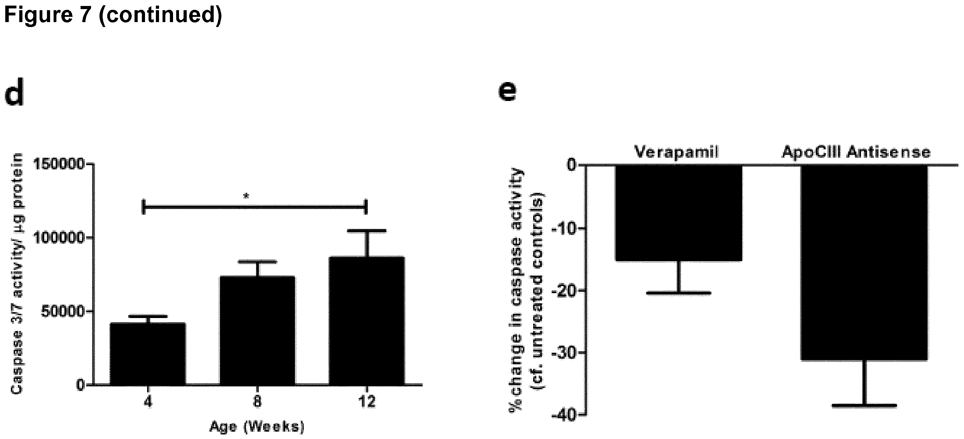

**Figure 7** shows the effect of apoCIII knockdown in 12-weeks old ob/ob islets. **(a)** Isolated islets were either directly lysed (ob/ob) or exposed to apoCIII-specific (AS)/inactive (Scr) antisense oligonucleotides for 18 h. ApoCIII expression was measured by real-time qPCR to determine the efficacy of apoCIII knockdown in cultured islets (n=4). **(b)** Ob/ob and ob/lean islets exposed to either active or inactive apoCIII antisense were loaded with Fura-2 and exposed to 11 mM glucose to determined glucose-stimulated Ca²⁺ influx (n=4-11). **(c)** 25 mM KCl was used to induce depolarization and subsequent Ca²⁺ influx (n=9-10). **(d)** Caspase 3/7 activity in ob/ob islets isolated from animals at 4-, 8- and 12-weeks of age (n=4). **(e)** Decrease in caspase activity in β- cells from 12-weeks old ob/ob mice either incubated overnight with the Ca²⁺- channel blocker verapamil (left column) or exposed to apoCIII antisense (right column) (n=4). Data presented as mean ± SEM. *P<0.05, ** P<0.01 by student's t-test or ANOVA (Tukey's post-hoc).

### DETAILED DESCRIPTION OF THE INVENTION

Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); PCR Protocols: A Guide to Methods and Applications (Innis, et al. 1990. Academic Press, San Diego, CA), Culture of Animal Cells: A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX).

As used herein, the singular forms "a", "an" and "the" include plural referents unless the context clearly dictates otherwise. "And" as used herein is interchangeably used with "or" unless expressly stated otherwise.

All embodiments of any aspect of the invention can be used in combination, unless the context clearly dictates otherwise.

In a first aspect, the present invention provides compositions comprising a compound of formula A-B, wherein A is a pancreatic β cell targeting moiety, and B is an inhibitor of Apolipoprotein CIII (apoCIII) or β1 integrin. In an additional aspect, the composition comprises a compound of formula A-B-C, wherein C is a compound that permits cell entry of the composition.

The inventors have surprisingly discovered that obesity is associated with a local production of apoCIII in pancreatic β-cells. The examples provided herein demonstrate that apoCIII is locally expressed and increased in islets from ob/ob mice that have reduced insulin signaling in their beta cells due to insulin resistance. As these islets contain > 90% β-cells, most of the apoCIII expression is derived from the insulin-secreting β-cells. Thus, the compositions of the inventon can be used, for example, in treating or limiting development of diabetes.

As used herein, an "inhibitor" of expression and/or activity of ApoCIII and/or β1 integrin includes compounds that reduce the transcription of ApoCIII and/or β1 integrin DNA into RNA, compounds that reduce translation of the ApoCIII and/or β1 integrin RNA into protein, and compounds that reduce the function of ApoCIII and/or β1 integrin protein. Such inhibiting can be complete inhibition or partial inhibition, such that the expression and/or activity of the ApoCIII and/or β1 integrin is reduced, resulting in a reduced ability to increase intracellular calcium concentration. Such inhibitors are selected from the group consisting of antibodies that bind to ApoCIII and/or β1 integrin; aptamers that can interfere with ApoCIII and/or β1 integrin activity; antisense oligonucleotides directed against the ApoCIII and/or β1 integrin protein, DNA, or mRNA; small interfering RNAs (siRNAs), short hairpin RNAs (shRNAs), microRNAs (miRNA) or small internally segmented interfering RNAs (sisiRNA) directed against the ApoCIII and/or β1 integrin protein, DNA, or mRNA, and any other chemical or biological compound that can interfere with ApoCIII and/or β1 integrin activity.

In various embodiments, the apoCIII inhibitor is selected from the group consisting of anti-apoCIII antibody, anti-apoCIII aptamer, apoCIII small interfering RNA, apoCIII small internally segmented interfering RNA, apoCIII short hairpin RNA, apoCIII microRNA, and apoCIII antisense oligonucleotides.

In various embodiments, the β1 integrin inhibitor is selected from the group consisting of anti-β1 integrin antibody, anti-β1 integrin aptamer, β1 integrin small interfering RNA, β1 integrin small internally segmented interfering RNA, β1 integrin short hairpin RNA, β1 integrin microRNA, and β1 integrin antisense oligonucleotides.

The pancreatic β cell targeting moieties of the compositions permit targeting of the composition to pancreatic β cells upon administration to a subject in need thereof. In some embodiments the pancreatic β targeting moiety can comprise an antibody, receptor, receptor ligand and the like, which preferentially and/or specifically bind to pancreatic β cells. One or more targeting moieties can be attached to the inhibitor to provide a composition that is capable targeting the composition to pancreatic β cells. In various embodiments, targeting moieties include, but are not limited to peptides that preferentially bind pancreatic β cells, antibodies that bind pancreatic β cells, and receptor ligands that bind pancreatic β cells.

In one embodiment, the pancreatic β cell specific targeting moiety comprises one or more peptides or other moieties that preferentially bind pancreatic β cells, selected from the group consisting of glucagon-like peptide-1 (GLP-1 SEQ ID NO: 73), glucagon-like peptide-2 (GLP-2 SEQ ID NO: 74), peptide YY (PYY SEQ ID NO: 3), neuropeptide Y (NPY SEQ ID NO: 4), pancreatic peptide (PPY SEQ ID NO: 5), exendin-4 (SEQ ID NO: 6), naphthylalanine and naphthylalanine derivatives (sequences shown in Table 1).

In other embodiments, the pancreatic β cell specific targeting moiety comprises a moiety that that selectively binds a pancreatic β cell protein selected from the group consisting of DiGeorge syndrome critical region gene 2 (DGCR2; SEQ ID NO: 7), golgi brefeldin A resistant guanine nucleotide exchange factor 1 (GBF1 SEQ ID NO: 8), orphan G protein-coupled receptor GPR44(GPR44 SEQ ID NO: 9), SerpinB10 (PI-10 SEQ ID NO: 10), FXYD domain containing ion transport regulator 2 (FXYD2 SEQ ID NO: 11), Tetraspanin-7 (TSPAN7 SEQ ID NO: 12), gap junction protein, delta 2, 36kDa (GJD2 SEQ ID NO: 13), solute carrier family 18 (vesicular monoamine), member 2 (SLC18A2 SEQ ID NO: 14), prokineticin receptor 1 (PROKR1 SEQ ID NO: 15), glutamate receptor, metabotropic 5 (GRM5 SEQ ID NO: 16), neuropeptide Y receptor Y2 (NPY2R SEQ ID NO: 17), glucagon-like peptide 1 receptor (GLP1R SEQ ID NO: 18), and transmembrane protein 27 (TMEM27 SEQ ID NO: 19).

**Table 1. Amino acid sequences.**

| Protein | Sequence |
|---|---|
| DiGeorge syndrome critical region gene 2 (DGCR2) | |
| Gene synonyms: DGS-C; IDD; LAN; SEZ-12 | |
| (SEQ ID NO: 7) | |
| golgi brefeldin A resistant guanine nucleotide exchange factor 1 (GBF1) | |
| Gene synonym: ARF1GEF | |
| (SEQ ID NO: 8) | |
| | |
| orphan G protein-coupled receptor GPR44 (GPR44) | |
| Gene synonym: GPR44 | |
| (SEQ ID NO: 9) | |
| SerpinB10 (PI-10) | |
| Gene synonyms: PI-10; PI10 | |
| (SEQ ID NO: 10) | |
| FXYD domain containing ion transport regulator 2 (FXYD2) | Isoform 1: |
| Gene synonyms: ATP1G1; HOMG2 | Isoform 2: (SEQ ID NO:72) |
| (SEQ ID NO: 11) | |
| Tetraspanin-7 (TSPAN7) | |
| Gene synonyms: A15; CCG-B7; CD231; DXS1692E; MRX58; MXS1; TALLA-1; TM4SF2; TM4SF2b (SEQ ID NO: 12) | |
| gap junction protein, delta 2, 36kDa (GJD2) | |
| Gene synonyms: CX36; GJA9 | |
| (SEQ ID NO: 13) | |
| solute carrier family 18 (vesicular monoamine), member 2 (SLC18A2) | |
| Gene synonyms: SVAT; SVMT; VAT2; VMAT2 | |
| (SEQ ID NO: 14) | |
| prokineticin receptor 1 (PROKR1) | |
| Gene synonyms: | |
| GPR73; GPR73a; PKR1; ZAQ | |
| (SEQ ID NO: 15) | |
| glutamate receptor, metabotropic 5 (GRM5) | |
| Gene synonyms: GPRC1E; mGlu5; MGLUR5 | |
| (SEQ ID NO: 16) | |
| neuropeptide Y receptor Y2 (NPY2R) | |
| Gene synonym: NPY2-R | |
| (SEQ ID NO: 17) | |
| glucagon-like peptide 1 receptor (GLP1R) | |
| Gene synonyms: GLP-1R; GLP-1-R; GLP-1 receptor | |
| (SEQ ID NO: 18) | |
| transmembrane protein 27 (TMEM27) | |
| Gene synonyms: NX-17; NX17 | |
| (SEQ ID NO: 19) | |
| glucagon (GCG) | |
| Gene synonyms: GLP1; GLP2; GRPP | |
| (SEQ ID NO: 1) | Glucagon-like peptide 1; GLP1 (aa92-128) (SEQ ID NO:73) HDEFERHAEGTFTSDVSSYLEGQAAKEFIAWLVKGRG |
| | Glucagon-like peptide 2; GLP2(aa146-178) (SEQ ID NO:74) HADGSFSDEMNTILDNLAARDFINWLIQTKITD |
| pancreatic peptide (PPY) | |
| Gene synonyms: PNP; PP | |
| (SEQ ID NO: 5) | |
| neuropeptide Y (NPY) | |
| Gene synonym: PYY4 | |
| (SEQ ID NO: 4) | |
| peptide YY (PYY) | |
| Gene synonyms: PYY-I; PYY1 | |
| (SEQ ID NO: 3) | |
| exendin-4 | |
| Gene synonym: (SEQ ID NO: 6) | |
| apolipoprotein C-III (APOC3) *Homo sapiens* | APOC3 Protein; SEQ ID NO.: 75 |
| Gene synonyms: HALP2; APOCIII | APOC3 Gene; SEQ ID NO.: 76 |

When the targeting moiety comprises an antibody, such antibodies can be polyclonal or monoclonal. The antibodies can be humanized, fully human, or murine forms of the antibodies. Such antibodies can be made by well-known methods, such as described in Harlow and Lane, Antibodies; A Laboratory Manual, Cold Spring Harbor Laboratory, Cold Spring Harbor, N.Y., (1988). In some embodiments, additional amino acid residues may be added to either the N- or C-terminus of the antibody or antibody fragment. When the targeting moiety comprises an aptamer, such aptamers can be oligonucleic acid or peptide molecules that bind to a specific target molecule. Methods of constructing and determining the binding characteristics of aptamers are well known in the art, and the aptamers can be isolated from random libraries or they can be previously identified peptides.

Attaching the pancreatic β cell targeting moiety to the inhibitor may be accomplished by any chemical reaction that will bind the two molecules so long as the pancreatic β cell targeting moiety and the inhibitor retain their respective activities. In one embodiment, both the β cell targeting moiety and the inhibitor comprise polypeptides, and the composition comprises a recombinant fusion protein. In another embodiment, both the β cell targeting moiety and the inhibitor comprise polynucleotides, and the composition comprises a recombinant nucleic acid. In other embodiments, a linkage between the pancreatic β cell targeting moiety and the inhibitor can include many chemical mechanisms, for instance covalent binding, affinity binding, intercalation, coordinate binding and complexation. Covalent binding can be achieved either by direct condensation of existing side chains or by the incorporation of external bridging molecules. Many bivalent or polyvalent linking agents are useful in coupling protein molecules, such as the antibodies, to other molecules. For example, representative, non-limiting examples of coupling agents can be organic compounds such as thioesters, carbodiimides, succinimide esters, disocyanates, glutaraldehydes, diazobenzenes and hexamethylene diamines.

In another embodiment, the compositions comprise a compound of general formula A-B-C, wherein C is a compound that permits cell entry of the composition. In one embodiment, the compound that permits cell entry of the composition may comprise a cell penetrating peptide. In other embodiments, the compound that permits cell entry of the composition may comprise a non-peptidic cell penetrating compound. Cell penetrating peptides or non-peptidic cell penetrating compounds facilitate uptake of the composition into the pancreatic β cells targeted by the targeting moiety of the composition. The cell penetrating compound can be linked to components A-B of the composition by any suitable technique that retains the activities of each component, as discussed herein. In one embodiment, each of A-B-C are polypeptides and the composition comprises a fusion protein.

Exemplary cell penetrating peptides include, but are not limited to RRASAP (SEQ ID NO: 20) LRRASAP (SEQ ID NO: 21) WLRRASAP (SEQ ID NO: 22) RRATAP (SEQ ID NO: 23) LRRATAP (SEQ ID NO: 24) WLRRATAP (SEQ ID NO: 25) RRAYAP (SEQ ID NO: 26) LRRAYAP (SEQ ID NO: 27) WLRRAYAP (SEQ ID NO: 28) RRADAP (SEQ ID NO: 29) LRRADAP (SEQ ID NO: 30) WLRRADAP (SEQ ID NO: 31) RRAEAP (SEQ ID NO: 32) LRRAEAP (SEQ ID NO: 33) WLRRAEAP (SEQ ID NO: 34) RRASAPRRASAP (SEQ ID NO: 35) LRRASAPLRRASAP (SEQ ID NO: 36) WLRRASAPWLRRASAP (SEQ ID NO: 37) RRATAPRRATAP (SEQ ID NO: 38) LRRATAPLRRATAP (SEQ ID NO: 39) WLRRATAPWLRRATAP (SEQ ID NO: 40) RRAYAPRRAYAP (SEQ ID NO: 41) LRRAYAPLRRAYAP (SEQ ID NO 42) WLRRAYAPWLRRAYAP (SEQ ID NO: 43) RRADAPRRADAP (SEQ ID NO: 44) LRRADAPLRRADAP (SEQ ID NO: 45) WLRRADAPWLRRADAP (SEQ ID NO: 46) RRAEAPRRAEAP (SEQ ID NO: 47) LRRAEAPLRRAEAP (SEQ ID NO: 48) WLRRAEAPWLRRAEAP (SEQ ID NO: 49), GRKKRRQRRRPPQ (SEQ ID NO:50); AYARAAARQARA (SEQ ID NO:51); DAATATRGRSAASRPTERPRAPARSASRPRRPVE (SEQ ID NO:52); GWTLNSAGYLLGLINLKALAALAKKIL (SEQ ID NO:53); PLSSISRIGDP (SEQ ID NO:54); AAVALLPAVLLALLAP (SEQ ID NO:55); AAVLLPVLLAAP (SEQ ID NO:56); VTVLALGALAGVGVG (SEQ ID NO:57); GALFLGWLGAAGSTMGAWSQP (SEQ ID NO:58); GWTLNSAGYLLGLINLKALAALAKKIL (SEQ ID NO:59); KLALKLALKALKAALKLA (SEQ ID NO:60); KETWWETWWTEWSQPKKKRKV (SEQ ID NO:61); KAFAKLAARLYRKAGC (SEQ ID NO:62); KAFAKLAARLYRAAGC (SEQ ID NO:63); AAFAKLAARLYRKAGC (SEQ ID NO:64); KAFAALAARLYRKAGC (SEQ ID NO:65); KAFAKLAAQLYRKAGC (SEQ ID NO:66), AGGGGYGRKKRRQRRR (SEQ ID NO:67); YGRKKRRQRRR (SEQ ID NO:68); and YARAAARQARA (SEQ ID 69).

The individual components of the composition can be made by any suitable means known to those of skill in the art. Once the composition has been formulated, it can be stored in sterile vials as a solution, suspension, gel, emulsion, solid, or as a dehydrated or lyophilized powder. Such formulations can be stored either in a ready-to-use form or in a form (e.g., lyophilized) requiring reconstitution prior to administration.

In a second aspect, the present invention provides an isolated nucleic acid encoding a fusion protein or recombinant nucleic acid of any embodiment or combination of embodiments of the compositions of the invention, operatively linked to a promoter. Thus, in some embodiments the fusion protein comprises a fusion protein of any embodiment or combination of embodiments of peptidic components A-B (and optionally C) of the compositions of the invention. In other embodiments, components A-B of the compositions of the invention are each polynucleotides, and thus the isolated nucleic acid encodes a recombinant nucleic acid composition of the invention. The isolated nucleic acid sequences may comprise additional sequences useful for promoting expression and/or purification of the encoded protein, including but not limited to polyA sequences, modified Kozak sequences, and sequences encoding epitope tags, export signals, and secretory signals, nuclear localization signals, and plasma membrane localization signals. The term "operatvely linked" is used herein to refer to an arrangement of flanking sequences wherein the flanking sequences so described are configured or assembled so as to perform their usual function. Thus, a flanking sequence operably linked to a coding sequence may be capable of effecting the replication, transcription, and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

In a third aspect, the present invention provides recombinant expression vectors comprising an isolated nucleic acid of the invention. The term "vector" as used herein refers to any molecule (*e.g.,* nucleic acid, plasmid, or virus) that is used to transfer coding information to a host cell. The term "vector" includes a nucleic acid molecule that is capable of transporting another nucleic acid to which it has been linked. One type of vector is a "plasmid," which refers to a circular double-stranded DNA molecule into which additional DNA segments may be inserted. Another type of vector is a viral vector, wherein additional DNA segments may be inserted into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) can be integrated into the genome of a host cell upon introduction into the host cell and thereby are replicated along with the host genome. In addition, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. The terms "plasmid" and "vector" may be used interchangeably herein, as a plasmid is the most commonly used form of vector. However, the invention is intended to include other forms of expression vectors, such as viral vectors (*e.g.,* replication defective retroviruses, adenoviruses, and adeno-associated viruses), which serve equivalent functions.

In a fourth aspect, the present invention provides recombinant host cells comprising an expression vector of any embodiment or combination of embodiments of the invention. The phrase "recombinant host cell" (or "host cell") as used herein refers to a cell into which a recombinant expression vector has been introduced. A recombinant host cell or host cell is intended to refer not only to the particular subject cell, but also to the progeny of such a cell. Because certain modifications may occur in succeeding generations due to either mutation or environmental influences, such progeny may not, in fact, be identical to the parent cell, but such cells are still included within the scope of the term "host cell" as used herein. A wide variety of host cell expression systems can be used to express the antibody-like binding proteins of the invention, including bacterial, yeast, baculoviral, and mammalian expression systems (as well as phage display expression systems). An example of a suitable bacterial expression vector is pUC19. To express an antibody-like binding protein recombinantly, a host cell is transformed or transfected with one or more recombinant expression vectors carrying DNA fragments encoding the polypeptide chains of the antibody-like binding protein such that the polypeptide chains are expressed in the host cell and, preferably, secreted into the medium in which the host cells are cultured, from which medium the antibody-like binding protein can be recovered.

The term "transformation" as used herein refers to a change in a cell's genetic characteristics, and a cell has been transformed when it has been modified to contain a new DNA. For example, a cell is transformed where it is genetically modified from its native state. Following transformation, the transforming DNA may recombine with that of the cell by physically integrating into a chromosome of the cell, or may be maintained transiently as an episomal element without being replicated, or may replicate independently as a plasmid. A cell is considered to have been stably transformed when the DNA is replicated with the division of the cell. The term "transfection" as used herein refers to the uptake of foreign or exogenous DNA by a cell, and a cell has been "transfected" when the exogenous DNA has been introduced inside the cell membrane. A number of transfection techniques are well known in the art. Such techniques can be used to introduce one or more exogenous DNA molecules into suitable host cells.

In a fifth aspect, the invention provides a composition comprising a compound of formula A-B, wherein A is a pancreatic β cell targeting moiety, and B is an inhibitor of expression and/or activity of Apolipoprotein CIII (apoCIII) and/or β1 integrin for use in treating or limiting development of diabetes in a subject. Thus, any embodiment or combination of embodiments of the compositions disclosed herein may be used in the present invention.

In various embodiments, the apoCIII inhibitor is selected from the group consisting of anti-apoCIII antibody, anti-apoCIII aptamer, apoCIII small interfering RNA, apoCIII small internally segmented interfering RNA, apoCIII short hairpin RNA, apoCIII microRNA, and apoCIII antisense oligonucleotides.

In various embodiments, the β1 integrin inhibitor is selected from the group consisting of anti-β1 integrin antibody, anti-β1 integrin aptamer, β1 integrin small interfering RNA, β1 integrin small internally segmented interfering RNA, β1 integrin short hairpin RNA, β1 integrin microRNA, and β1 integrin antisense oligonucleotides.

In various embodiments, the pancreatic β cell specific targeting moiety comprises a moiety that that selectively binds a protein selected from the group consisting of DiGeorge syndrome critical region gene 2 (DGCR2), golgi brefeldin A resistant guanine nucleotide exchange factor 1 (GBF1), orphan G protein-coupled receptor GPR44(GPR44), SerpinB10 (PI-10), FXYD domain containing ion transport regulator 2 (FXYD2), Tetraspanin-7 (TSPAN7), gap junction protein, delta 2, 36kDa (GJD2), solute carrier family 18 (vesicular monoamine), member 2 (SLC18A2), prokineticin receptor 1 (PROKR1), glutamate receptor, metabotropic 5 (GRM5), neuropeptide Y receptor Y2 (NPY2R), glucagon-like peptide 1 receptor (GLP1R), and transmembrane protein 27 (TMEM27). In further embodiments, the pancreatic β cell specific targeting moiety comprises a moiety selected from the group consisting of glucagon-like peptide-1 (GLP-1), glucagon-like peptide-2 (GLP-2), peptide YY (PYY), neuropeptide Y (NPY), pancreatic peptide (PP), exendin-4, naphthylalanine and naphthylalanine derivatives.

As used herein, "pancreatic β cells" are any population of cells that contains pancreatic β islet cells. Such pancreatic β islet cell populations include the pancreas, isolated pancreatic islets of Langerhans ("pancreatic islets") and isolated pancreatic β islet cells.

In one embodiment, the composition is for use in treating diabetes. In this embodiment, the subject has been diagnosed with type 1 or type 2 diabetes. As used herein, "diabetes" is characterized by insufficient or no production of insulin by the pancreas, leading to high blood sugar levels.

As used herein, "treating diabetes" means accomplishing one or more of the following: (a) reducing the severity of the diabetes or diabetic complications; (b) limiting or preventing development of diabetic complications; (c) inhibiting worsening of diabetic complications or of symptoms characteristic of diabetes; (d) limiting or preventing recurrence diabetic complications or of symptoms characteristic of diabetes; (e) limiting or preventing recurrence of diabetic complications or of symptoms characteristic of diabetes in patients that were previously symptomatic.

Symptoms characteristic of diabetes that can be treated by the compositions for use of the invention include, but are not limited to, elevated blood glucose levels, decreased insulin production, insulin resistance, proteinuria, and impaired glomerular clearance. Diabetic complications that can be treated according to the compositions for use of the invention include, but are not limited to, complications in the nerves (such as diabetic neuropathy) and complications associated with smooth muscle cell dysregulaton (including but not limited to erectile dysfunction, bladder dysfunction, and vascular complications including but not limited to atherosclerosis, stroke, and peripheral vascular disease)

In another embodiment, the composition is for use in limiting development of diabetes. In this aspect, the subject is at risk of type 1 or type 2 diabetes, and a benefit is to limit development of diabetes and/or diabetic complications. Any subject at risk of developing diabetes can be treated, including but not limited to subjects with one or more of, metabolic syndrome, known genetic risk factors for diabetes, a family history of diabetes, and obesity.

In a further embodiment, the compositions for use in treating or limiting development of diabetes and/or diabetic complications further comprises treating those individuals that have been identified as overexpressing apoCIII compared to control. Increases in apoCIII expression precede development of diabetic complications, and thus this embodiment permits early detection of suitable patients for treatment using the methods of the invention.

As used herein, "overexpression" is any amount of apoCIII expression above control. Any suitable control can be used, including apoCIII expression levels from a subject known not to be suffering from diabetes, or previously determined standardized expression levels of apoCIII from a population of similar patient samples. Any amount of increased apoCIII expression relative to control is considered "overexpression"; in various embodiments, the overexpression comprises at least 10%, 20%, 50%, 100%, 200%, or greater increased apoCIII expression compared to control. In a preferred embodiment, apoCIII expression is detected in blood or serum samples. In one embodiment to evaluate the levels of apoCIII in sera, albumin is removed from serum samples using standard techniques, such as via use of Montage Albumin Deplete Kit (Millipore) or AlbuSorb™ (Biotech Support Group). The collected sera samples can then be freeze-dried overnight and run on sep-Pak C18. The eluted proteins can be freeze-dried and thereafter dissolved in 100 µL 0.1 % TFA and run on an ACE C18 10- × 0.21-cm column 20-60%, and the area under the curve, where apoCIII elutes, evaluated. ApoCIII can be identified using any suitable technique, including but not limited to MALDI mass spectrometry.

As used herein, the term "subject" or "patient" is meant any subject for which therapy is desired, including humans, cattle, dogs, cats, guinea pigs, rabbits, rats, mice, insects, horses, chickens, and so on. Most preferably, the subject is human.

The therapeutic may be administered by any suitable route, including but not limited to oral, topical, parenteral, intranasal, pulmonary, or rectal in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a compound of the invention and a pharmaceutically acceptable carrier. The therapeutic may be present in association with one or more non-toxic pharmaceutically acceptable carriers and/or diluents and/or adjuvants, and if desired other active ingredients. The therapeutic may be in a form suitable for oral use, for example, as tablets, troches, lozenges, aqueous or oily suspensions, dispersible powders or granules, emulsion, hard or soft capsules, or syrups or elixirs.

In another embodiment, the composition can also be administered locally via implantation of a membrane, sponge, or other appropriate material onto which the composition of the invention has been absorbed or encapsulated. Where an implantation device is used, the device can be implanted into or next to the pancreas, and delivery of the desired molecule can be via diffusion, timed-release bolus, nano-containers or continuous administration.

The dosage range depends on the choice of the compound, the route of administration, the nature of the formulation, the nature of the subject's condition, and the judgment of the attending practitioner. For example, oral administration would be expected to require higher dosages than administration by intravenous injection. Variations in these dosage levels can be adjusted using standard empirical routines for optimization, as is well understood in the art

Acceptable formulation materials preferably are nontoxic to recipients at the dosages and concentrations employed. The therapeutic composition can contain formulation materials for modifying, maintaining, or preserving, for example, the pH, osmolarity, viscosity, clarity, color, isotonicity, odor, sterility, stability, rate of dissolution or release, adsorption, or penetration of the composition. Suitable formulation materials include, but are not limited to, amino acids (such as glycine, glutamine, asparagine, arginine, or lysine), antimicrobials, antioxidants (such as ascorbic acid, sodium sulfite, or sodium hydrogen-sulfite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as polysorbate 20 or polysorbate 80; triton; tromethamine; lecithin; cholesterol or tyloxapal), stability enhancing agents (such as sucrose or sorbitol), tonicity enhancing agents (such as alkali metal halides - preferably sodium or potassium chloride - or mannitol sorbitol), delivery vehicles, diluents, excipients and/or pharmaceutical adjuvants (see, e.g., REMINGTON'S PHARMACEUTICAL SCIENCES (18th Ed., A.R. Gennaro, ed., Mack Publishing Company 1990), and subsequent editions of the same).

The therapeutic compositions will be determined by a skilled artisan depending upon, for example, the intended route of administration, delivery format, and desired dosage. Such compositions can influence the physical state, stability, rate of *in vivo* release, and rate of *in vivo* clearance of the antibody-like binding protein.

### EXAMPLES

### MATERIAL AND METHODS

**Animals.** Age-matched ob/ob (Lep^{ob}/Lep^{ob}) and ob/lean (Lep^{ob}/Lep⁺) on a C57BL/6J background were obtained from a breeding colony at Karolinska Institutet, Stockholm, Sweden. Mice used in experiments were genotyped. All experiments were performed on mice, between 4 to 12 weeks of age. The animals were housed in a temperature- and humidity-controlled room with 12-hour lightdark cycles, regular chow and water *ad libitum.* Animal care and experimentations were carried out according to the Animal Experiment Ethics Committee at Karolinska Institutet.

**Cell Culture.** MIN6-m9 cells, between passage 35 and 42, were cultured in DMEM containing 11.1 mM glucose and supplemented with 100 U/ml penicillin, 100 µg/ml streptomycin, 2 mM glutamine, 10% FCS, and 75 µM β-mercaptoethanol at 5% CO₂ and 37°C. MIN6 cells were transfected using Lipofectamine (Invitrogen, USA) with the control expression construct containing tdTomato or c-Myc tagged mouse apoCIII construct for 24 hours. All experiments were performed 48 h after transfection.

**Metabolic Studies.** Non-fasted glucose was measured with a glucose meter (Accu-Chek™ Advantage, Roche Diagnostics, USA) and serum insulin by the ArcDia™ 2-photon fluorescence excitation microparticle fluorometry (TPX) assay for insulin (ArcDia Group, Finland).

**Pancreatic islet isolation.** Animals were euthanized by cervical dislocation and the pancreas perfused with 3 mL of 1 mg/mL collagenase A (Roche, USA) in Hank's balanced salt solution (HBSS) (Sigma, Sweden) buffer supplemented with 0.2 % BSA and 25 mM HEPES buffer. Pancreas was thereafter extracted and digested at 37°C for 20 min. Islets were handpicked and were either immediately used for mRNA/ protein analysis or were cultured overnight in RPMI 1640 medium supplemented with 10 % FCS, 2 mM glutamine as well as 100 U/ml and 100 µg/ml of penicillin and streptomycin, respectively.

**Dispersion of pancreatic islets into single cells.** Isolated islets were washed with HBSS without Ca²⁺ and Mg²⁺ and dispersed into single cells by incubation with Accutase in PBS containing 0.5mM EDTA (Innovative Cell Technologies, Cytotech, Denmark). Cells were kept free floating in Petri dishes in the same medium as for islets.

**RNA isolation and quantitative RT-PCR analysis.** Total RNA was isolated from sorted cells or isolated pancreatic islets using the RNAeasy™ Micro Kit (Qiagen, Germany). Briefly, cells were lysed by first using RLT lysis buffer followed by a spin Qiashredder™ lysis. For liver and intestine RNA isolation, tissue/cell disruption was first carried out by a hand-held rotor-stator homogenizer in RLT lysis buffer followed by 1000 x g centrifugation for 5 minutes. The supernatant was subsequently transferred to a Qiashredder™ column to complete the lysis prior to the RNAeasy™ Mini Kit application (Qiagen, Germany). All lysates were applied to the RNAeasy™ spin column and the subsequent RNA isolation and on column DNAse treatment were carried out according to manufacturer recommendations. Total RNA was reverse transcribed at 37° C with Multiscribe™ (Applied Biosystems, USA). The expression of all genes was measured by real-time quantitative PCR (qPCR) with Taq™ SYBR Supermix with ROX (Invitrogen, USA) on an ABI7900HT instrument (Applied Biosystems, USA). β-action was used as an endogenous control with 1 cycle at 95° C for 10 min followed by 40 cycles of tandem 95° C for 30 seconds and 60° C for 1 minute. In all cases, unless otherwise stated, gene-specific intron spanning primers were used and the PCR melting curve produced one single peak corresponding to a specific single amplified product.

**Western Blotting.** Islets or cells were washed with PBS, lysed with RIPA buffer (50 mM Tris pH 7.5, 150 mM NaCl, 1 % NP-40, 0.5 % Na deoxycholate, 0.1 % SDS, EDTA-free protease inhibitor cocktail, 1 µg/ml pepstatine and leupeptin). Lysates were passed 5 times through a syringe needle (0.33 x13 mm/29 Gx1/2) followed by 30 minutes incubation on a rotator at 4° C. Homogenates were spun at 10,000 x g for 10 minutes and the protein amount was determined in the supernatants by the BCA method (Pierce). Equal amounts of protein (25-50 µg) were separated over a 4-12 % Bis-Tris gel with MES buffering system (Invitrogen, USA). Proteins were subsequently electrotransferred to PVDF membrane. In case of the phosphospecific antibodies, the membranes were probed with the respective antibodies and then stripped and reprobed with antibodies recognizing the respective total protein levels. Rabbit anti-FoxO1, rabbit anti-phospho-FoxO1, Akt and phospho-Akt antibody were purchased from Cell Signaling Technology (Danvers, USA). Further antibodies used were rabbit anti-apoCIII antibody (Santa-Cruz, USA), guinea-pig c-peptide (Abcam, UK), mouse anti-cMyc (Santa Cruz, USA) and mouse anti-tubulin (Abcam, UK). Immunoreactivity was detected with horseradish peroxidase-conjugated secondary antibodies using the ECL system (Amersham, USA).

**Immunocytochemistry.** To visualize Myc-tagged apoCIII in MIN6 cells, post-transfected cells were cultured onto glass cover slips prior to 3% PFA fixation for 30 minutes. Subsequently, cells were incubated in antibodies specific for c-Myc (mouse monoclonal, Santa-Cruz, USA) and C-peptide (rabbit polyclonal, Cell Signaling, USA) in 10% Goat serum blocking buffer, overnight at 4°C with gentle shaking. Cells were incubated with Alexa 488 anti-mouse (Santa-Cruz, USA) and Alexa 546 anti-rabbit (Cell Signaling, USA) in blocking buffer for 1 hour followed by washing and mounting using VectaShield™ mounting media with DAPI (Vector Labs, USA). The human islets were fixed in 4% paraformaldehyde for 15 minutes, washed in PBS and incubated in 20% sucrose/PBS overnight at 4°C. Thereafter they were embedded in Tissue-Tek® OCT™ (Sakura, The Netherlands) and cryosectioned (10 µm). The sections were permeabilized in 0.3% Triton-X/PBS for 15 minutes, treated with 3% H₂O₂ block for 10 minutes and washed in PBS and subsequently blocked in 10% goat serum. Immunohistochemical staining was performed using goat anti-human apoCIII (1:100, Academy Biomedical, USA) overnight incubation at 4°C, followed by Alexa™ Flour 488-labeled rabbit anti-goat (1:1000, Invitrogen) for 30 minutes at room temperature. Counterstaining was performed with DAPI (1:1000; Sigma-Aldrich). Following the above-described method, a blockage of goat anti-human apoCIII was also performed as a control by using the goat anti-human apoCIII pretreated over night at 4°C with apoCIII protein (immunizing peptide).

**Measurements of [Ca²⁺]ᵢ.** Changes in [Ca²⁺]ᵢ were recorded in islets after a 16 hour incubation period with 0.07 mg/ml apoCIII antisense or an inactive control (Isis Pharmaceuticals, USA) in RPMI 1640 medium. The basal medium used for islet perifusion experiments was a HEPES buffer (pH 7.4), containing: 125 mM NaCl, 5.9 mM KCl, 2.6 mM CaCl₂, 1.2 mM MgCl₂, and 25 mM HEPES, 0.1% BSA supplemented with either 3 mM glucose, 11 mM glucose or 25 mM KCl. Islets were attached to coverslips using Puramatrix™ Hydrogel (BD Biosystem, USA), loaded with 2 µM fura-2 acetoxymethyl ester (Molecular Probes, USA) and mounted on an inverted epifluorescence microscope (Zeiss, Axiovert™ 135) connected to a Spex Industries Fluorolog system for dual-wavelength excitation fluorimetry. The emissions due to the two excitation wavelengths of 340 and 380 nm were used to calculate the fluorescence ratio 340/380, reflecting changes in [Ca²⁺]ᵢ. To compensate for possible variations in output of light intensity from the two monochromators, each experiment also included measurement of a 360/360 ratio. Every experiment was normalized by dividing all fluorescence ratios by the corresponding 360/360 ratio.

**Caspase assay.** Activity of capase 3/7 was determined using SensoLyte™ Homogeneous Rh110 Caspase 3/7 assay kit according to manufacturer's instructions, with modifications (AnaChem, USA). Briefly, islets or cells were harvested, lysed using lysis buffer (AnaChem, USA) and the protein quantified using BCA method. 10 µg of protein was loaded in a black 384-well plate and topped up with the appropriate amount of assay buffer (AnaChem, USA) containing Rh110 Caspase 3/7 Substrate. Plate was incubated in the dark for 1 hour at 24°C. Fluorescence intensity at Ex/Em = 490/520nm was measured to determine the relative caspase activity.

**RNA analysis.** 5'- RACE was performed by using ob/ob islets (1.5 µg RNA) Ambion FirstChoice™ RACE-ready cDNA kit (Invitrogen, USA). PCR was carried-out following manufacturer's instructions. Platinum *Taq* polymerase (Invitrogen, USA) was used in RACE-PCR. Primer sequences for apoCIII-outer (primary amplification): 5' GGAGGGGTGAAGACATGAGA -3' (SEQ ID NO: 70); apoCIII-inner (nested amplification): 5' TCTGAAGTGATTGTCCATCCAG -3' (SEQ ID NO: 71). An aliquot of the first PCR (outer PCR) was used for subsequent nested PCR (inner PCR). Amplified cDNA were gel purified and subcloned into the pCRII-TOPO vector (Invitrogen, USA) and sequenced with M13 reverse and M13 forward primers.

**Statistical analysis.** Studies were repeated at least three times. For individual experiment, the number of animals used (n) is included in each figure legend in parenthesis. All results are expressed as mean ± SEM (indicated by error bars). Statistical analyzes were performed with either GraphPad™ Prism 5 or Microsoft Excel 2007. A student's t-test or one-way ANOVA (Tukey's post-hoc) were used as appropriate. P values <0.05 were considered statistically significant.

### Example 1. Insulin signaling in ob/ob islets.

To determine the changes in insulin signaling in ob/ob mouse islets, which consist of more than 90% β-cells, the phenotypic changes were tracked in these mice at 4, 8 and 12 weeks of age, measuring parameters such as body weight, non-fasting blood glucose and insulin concentrations. Age-matched ob/lean mice that were routinely genotyped from 4 weeks onwards were used as controls. Since the ob/ob mouse is a known transient hyperglycemia model with increasing hyperglycemia up to 14 weeks of age, the study was limited to the first 12 weeks of age and found that both weight and non-fasting blood glucose levels progressively increased from 4- to 12-weeks of age (Fig. 1a and Fig 1b). However, the non-fasting insulin levels in the ob/ob mice remained higher than in control animals (Fig. 1c).

With prolonged hyperinsulinemia, insulin signaling at the islet level may be compromised, similar to what is seen in the β-cell-specific insulin receptor knockout mouse (βIRKO) and the β-cell-specific PI3K subunit p85 β knockout mouse (βPik3r1). The mRNA expression levels of several genes known to be involved downstream of islet insulin signaling was determined. The expression of *gck, irs1*, *irs2*, *vamp2, snap25* and *rab27a* were shown to be controlled by insulin receptor (IR) activated phosphatidylinositol 3-kinase (PI3K) activity. All genes were down-regulated in 12 weeks old ob/ob islets (Fig. 1d). This is consistent with previous findings where a significantly reduced PI3K activity was found in 12-weeks old ob/ob islets as compared with their age-matched ob/lean littermates.

### Example 2. Progressive change of FoxO1-regulated gene expression in ob/ob islets over time.

One of the ways in which insulin induces changes in gene expression is through the phosphorylation cascade of Akt and its downstream target forkhead transcription factor FoxO1. Insulin-mediated activation of Akt leads to the phosphorylation of FoxO1 with its nuclear exclusion and loss of transcriptional activity. This signaling cascade has been shown to be crucial in maintaining β-cell function and proliferation during either insulin resistance or insult/injury. Levels of phospho-Akt and phospho-FoxO1 in ob/ob islets were determined at 4-, 8- and 12-weeks of age. There was a significant reduction in phospho-Akt (Ser473) and phospho-FoxO1 (Ser256) in the 12-weeks old ob/ob islet as compared to the islets at 4-weeks of age (Fig. 2a). Total Akt protein levels within these islets were not significantly different across time. Messenger RNA levels of FoxO1 were determined, and found no significant difference in all the age groups studied. The reduction in phospho-FoxO1 further corroborates the changes in ob/ob islet gene expression since *iss1, irs2*, *vamp2* and *rab27a* have been suggested to be repressed by FoxO1.

Insulin receptor-mediated repression of FoxO1 is pivotal in maintaining the balance between carbohydrate and fat metabolism in hepatocytes. Reduced FoxO1 phosphorylation, and subsequent activation, in insulin-resistant hepatocytes promote the expression of apoCIII, a modulator of circulating triglycerides, glucose 6 phosphatase a key gluconeogenic enzyme, and mttp1, a microsomal triglyceride transfer protein that is involved in lipoprotein assembly. To explore if a similar mechanism occurs in ob/ob islets with declining levels of phospho-FoxO1, the mRNA expression levels of Foxo1 target genes apoCIII, g6pc2 (islet specific glucose-6-phosphatase) and mttp1 were determined. There was a significant increase in ob/ob islet mRNA expression of all three genes across time (Fig. 2b). Together, these data suggest that insulin resistance at the islet level leads to a change in the expression of genes that are positively or negatively regulated by FoxO1. Of these, apoCIII expression stands out as a novel islet factor that has been shown to disrupt β-cell function. G6pc2 was originally identified as a variant in T2DM genome wide association (GWA) studies but its function did not appear to translate into an increased risk of T2DM.

There was no significant difference in liver and intestine apoCIII expression in ob/ob mice at the age-groups studied (Fig. 3). The systemic levels of apoCIII in ob/ob mice were also examined, and it was surprisingly discovered that the apoCIII immunoblots showed increasing apoCIII levels in the serum of ob/ob mice from 4 to 12 weeks of age (Fig. 1c). As liver apoCIII accounts for the bulk of circulating apoCIII, the higher levels of circulating apoCIII could be the result of reduced cellular uptake of triglyceride-rich (VLDL) remnants rather than increased production of VLDL particles.

### Example 3. ApoCIII is detected in both ob/ob mice and human pancreatic islets.

The presence of apoCIII in pancreatic islet has not been observed before and is interesting since this apolipoprotein is known to be involved in both β-cell dysfunction and T1DM. A proteomic characterization of single pancreatic islets showed presence of apoCIII. However, this can be the result of either systemic circulating apoCIII residing within the microvasculature of pancreatic islets or, as shown here, a local production of apoCIII within the islet itself. Comparatively, the expression of apoCIII in ob/lean pancreatic islets is about 3000-fold lower compared to the liver (Fig. 4a). The small intestine, the other known organ expressing apoCIII, has an expression that is 10-fold lower compared to the liver, but 300-fold higher compared to islet apoCIII expression. Nevertheless, a three-fold increase in apoCIII mRNA seen in the 12-weeks old ob/ob islet may exert profound autocrine/ paracrine effects within the islet (Fig. 2b). A 5'RACE of apoCIII transcripts in islets of 12-weeks old ob/ob mice conformed the presence of two transcripts with a differing 5'-untranslated region and a 100 % identical coding region (Fig. 5). The predominant islet apoCIII transcript is the same transcript that is expressed in the liver, suggesting that the same promoter is governing apoCIII transcription in the liver and in islet cells.

The source of apoCIII within the islets was determined. The endocrine portion of the ob/ob islet is made up of approximately 90 % β-cells, making this cell type a prime candidate for apoCIII expression. To examine this, a flow cytometry protocol originally optimized to sort out rat β-cells from the total pancreatic islet cell milieu was used. Subsequent characterization of sorted β-cells revealed enriched expression of insulin, low expression of somatostatin and undetectable expression of glucagon and pancreatic polypeptide (Fig. 4b). The expression of apoCIII was similarly higher, albeit lower compared to insulin, in the β-cell enriched population strongly suggesting that apoCIII is expressed in the pancreatic β-cell (Fig. 4b). Immunofluorescence staining using human apoCIII antibodies revealed the presence of apoCIII in human islet preparations from five donors, three males and two females, with no history of diabetes (Fig. 4c). Hence, these data demonstrate that the local production of apoCIII in pancreatic islets is conserved from rodents to humans.

### Example 4. ApoCIII overexpression increases [Ca²⁺]ᵢ in MIN6 cells.

Exogenously added human apoCIII induces apoptosis in the rat insulin secreting cells lines RINm5F and INS-1E and in primary mouse β-cells. It was therefore important to determine if a local production of apoCIII likewise affected mouse β-cell function and apoptosis. Hampered by the ability of the antibody to recognize apoCIII in mouse liver sections and lysates, a c-Myc tagged mouse apoCIII construct was used that allows determining the localization of Myc-apoCIII within β-cells. Myc-apoCIII was detectable in media of the Myc-apoCIII-transfected cells after a 24-hour culture, indicating that the synthesized apoCIII is being released into the extracellular medium (Fig. 6a). No co-localization between Myc-apoCIII and insulin C-peptide could be detected in the transfected cells (Fig. 6b), suggesting that apoCIII is consecutively released from the β-cell and not co-released with insulin. MIN6 cells transfected with either Myc-apoCIII or tdTomato (control vector) were loaded with Fura-2AM after 48 hours to determine the effect of mouse apoCIII on [Ca²⁺]ᵢ upon KCl stimulation. An increase in [Ca²⁺]ᵢ after KCl exposure was observed in MIN6 cells expressing Myc-apoCIII as compared to the control transfected cells (Fig. 6c). This was consistent with previous effects of exogenously added human apoCIII on both rat insulinoma cells and primary β-cells, indicating a conservation of function between human apoCIII and mouse apoCIII despite only having a 60% amino acid identity. The transfection efficiency was about 50%, but a higher increase in [Ca²⁺]ᵢ, upon depolarization was seen in non-transfected cells in the same culture dish, and the levels were above those from control transfected cells. This suggests that the non-transfected cells were affected by the apoCIII released into the media from neighboring Myc-apoCIII expressing cells.

### Example 5. Reduced endogenous islet apoCIII results in improved [Ca²⁺]ᵢ handling and less apoptosis.

To explore the effect of increased endogenous apoCIII expression within islets from 12-weeks old ob/ob mice, knockdown experiments using antisense oligonucleotides specific for apoCIII and an inactive control were performed. The expression of apoCIII in the islets exposed to antisense nucleotides for 18-hours was 20% compared to control islets (Fig. 7a). The overall expression of apoCIII in pancreatic islets fell to about 50% just after 18 hours in culture compared to mRNA levels of apoCIII in freshly isolated islets, suggesting that an artificial milieu associated with islet culture. Subsequently pancreatic islets were loaded with Fura-2AM to determine the effect of apoCIII on [Ca²⁺]ᵢ after both high glucose and KCl exposure. Ob/ob islets were more responsive to high glucose concentrations, as compared to ob/lean islets, with a significantly higher peak amplitude of [Ca²⁺]ᵢ (Fig. 7b). The knockdown of apoCIII in 12-weeks old ob/ob islets reduced the peak amplitude of [Ca²⁺]ᵢ upon both high glucose and KCl stimulation (Fig. 7b, 7c).

It was determined if reducing endogenous apoCIII affects apoptosis in ob/ob islets. Although ob/ob islets are hyperplastic with massive β-cell proliferation, it does not rule out the presence of apoptosis in these islets; therefore the level of apoptosis was determined in freshly isolated islets of ob/ob mice at weeks 4-, 8- and 12 (when phospho-FoxO1 declines) by measuring the activity of caspase 3/7. Apoptosis in ob/ob islets was significantly higher in 12-weeks old ob/ob islets as compared to islets at 4 weeks of age (Fig. 7d). Lowering endogenous apoCIII in 12-weeks-old ob/ob islets, when apoCIII levels are the highest, decreased caspase 3/7 activity, as a measure of apoptosis, by 31 % compared to islets treated with scrambled antisense (Fig. 7e). Previous results have shown that apoCIII increases the activity of the voltage- gated Ca²⁺-channel. Exposing β-cells to the Ca²⁺-channel blocker verapamil reduced caspase activity by 15% (Fig. 7e), suggesting that increased FoxO1-induced expression of apoCIII within the ob/ob pancreatic islet leads to unbalanced [Ca²⁺]ᵢ and an increase in β-cell apoptosis.

These results show that obesity is associated with a local production of apoCIII in pancreatic β-cells and that increased levels of apoCIII leads to insulin resistance, increased [Ca2+]ᵢ and β-cell apoptosis; therefore identifying local islet production of apoCIII as a novel factor for mediating β-cell apoptosis in insulin resistant states of T2DM. The effects are reversible as reducing endogenouse β-cell apoCIII normalizes [Ca2+]ᵢ and improves β-cell survival. Hence, locally enhanced apoCIII can interfere with β-cell function and survival and have an impact on development of the T2DM phenotype. The results demonstrate that apoCIII is locally expressed and increased in islets from ob/ob mice that have reduced insulin signaling. As these islets contain > 90% β-cells, most of the apoCIII expression is derived from the insulin-secreting β-cells. ApoCIII induced an increase in cytoplasmic free Ca2+ concentration and β-cell apoptosis that was abrogated by lowering apoCIII levels or blocking the voltage-gated Ca2+-channels. This makes apoCIII a prime target that links β-cell insulin resistance to reduction in β-cell mass in T2DM. Consequently, inhibition of β-cell apoCIII expression constitutes a novel mechanism to maintain β-cell function and survival.

In summary, insulin signaling in β-cells is essential to keep apoCIII expression low. When insulin signaling is impaired at the islet level, FoxO1 translocation into the nucleus induces changes in gene expression which include, among other alterations, a promotion of apoCIII transcription. The increase in local production of apoCIII subsequently leads to an increase in [Ca²⁺]ᵢ which in turn results in an increase in β-cell apoptosis. Hence, preventing insulin resistance at the islet level is crucial to preserve β-cell mass, especially during the late phase of T2DM where β-cells are progressively lost. Islet apoCIII is a key player in the promotion of β-cell apoptosis and lowering local β-cell production of apoCIII may provide a new therapeutic for T2DM.

### REFERENCES

Martin, B.C., et al. Role of glucose and insulin resistance in development of type 2 diabetes mellitus: results of a 25-year follow-up study. Lancet 340, 925-929 (1992).
Lillioja, S., et al. Insulin resistance and insulin secretory dysfunction as precursors of non-insulin-dependent diabetes mellitus. Prospective studies of Pima Indians. The New England journal of medicine 329, 1988-1992 (1993).
Kahn, C.R. Banting Lecture. Insulin action, diabetogenes, and the cause of type II diabetes. Diabetes 43, 1066-1084 (1994).
Leibiger, I.B., Leibiger, B., Moede, T. & Berggren, P.O. Exocytosis of insulin promotes insulin gene transcription via the insulin receptor/PI-3 kinase/p70 s6 kinase and CaM kinase pathways. Molecular cell 1, 933-938 (1998).
Kulkarni, R.N., et al. Tissue-specific knockout of the insulin receptor in pancreatic beta cells creates an insulin secretory defect similar to that in type 2 diabetes. Cell 96, 329-339 (1999).
Kulkarni, R.N., et al. Altered function of insulin receptor substrate-1-deficient mouse islets and cultured beta-cell lines. J Clin Invest 104, R69-75 (1999).
Okada, T., et al. Insulin receptors in beta-cells are critical for islet compensatory growth response to insulin resistance. Proceedings of the National Academy of Sciences of the United States of America 104, 8977-8982 (2007).
Ueki, K., et al. Total insulin and IGF-I resistance in pancreatic beta cells causes overt diabetes. Nature genetics 38, 583-588 (2006).
Hashimoto, N., et al. Ablation of PDK1 in pancreatic beta cells induces diabetes as a result of loss of beta cell mass. Nature genetics 38, 589-593 (2006).
Kaneko, K., et al. Class IA phosphatidylinositol 3-kinase in pancreatic beta cells controls insulin secretion by multiple mechanisms. Cell metabolism 12, 619-632 (2010).
Krauss, R.M., Herbert, P.N., Levy, R.I. & Fredrickson, D.S. Further observations on the activation and inhibition of lipoprotein lipase by apolipoproteins. Circ Res 33, 403-411 (1973).
Ginsberg, H.N., et al. Apolipoprotein B metabolism in subjects with deficiency of apolipoproteins CIII and AI. Evidence that apolipoprotein CIII inhibits catabolism of triglyceride-rich lipoproteins by lipoprotein lipase in vivo. J Clin Invest 78, 1287-1295 (1986).
Chen, M., Breslow, J.L., Li, W. & Leff, T. Transcriptional regulation of the apoC-III gene by insulin in diabetic mice: correlation with changes in plasma triglyceride levels. J Lipid Res 35, 1918-1924 (1994).
Altomonte, J., et al. Foxo1 mediates insulin action on apoC-III and triglyceride metabolism. J Clin Invest 114, 1493-1503 (2004).
Caron, S., et al. Transcriptional activation of apolipoprotein CIII expression by glucose may contribute to diabetic dyslipidemia. Arteriosclerosis, thrombosis, and vascular biology 31, 513-519(2011).
Salerno, A.G., et al. Overexpression of apolipoprotein CIII increases and CETP reverses diet-induced obesity in transgenic mice. Int J Obes (Lond) 31, 1586-1595 (2007).
Waterworth, D.M., et al. ApoCIII gene variants modulate postprandial response to both glucose and fat tolerance tests. Circulation 99, 1872-1877 (1999).
Petersen, K.F., et al. Apolipoprotein C3 gene variants in nonalcoholic fatty liver disease. The New England Journal of Medicine 362, 1082-1089 (2010).
Juntti-Berggren, L., et al. Apolipoprotein CIII promotes Ca2+-dependent beta cell death in type 1 diabetes. Proceedings of the National Academy of Sciences of the United States of America 101, 10090-10094 (2004).
Refai, E., et al. Transthyretin constitutes a functional component in pancreatic beta-cell stimulus-secretion coupling. Proceedings of the National Academy of Sciences of the United States of America 102, 17020-17025 (2005).
Holmberg, R., et al. Lowering apolipoprotein CIII delays onset of type 1 diabetes. Proceedings of the National Academy of Sciences of the United States of America 108, 10685-10689 (2011).
Hellman, B. Studies in obese-hyperglycemic mice. Annals of the New York Academy of Sciences 131, 541-558. (1965).
Coleman, D.L. & Hummel, K.P. The influence of genetic background on the expression of the obese (Ob) gene in the mouse. Diabetologia 9, 287-293 (1973).
Huo, L., et al. Leptin-dependent control of glucose balance and locomotor activity by POMC neurons. Cell metabolism 9, 537-547 (2009).
Kieffer, T.J., Heller, R.S., Leech, C.A., Holz, G.G. & Habener, J.F. Leptin suppression of insulin secretion by the activation of ATP-sensitive K+ channels in pancreatic beta-cells. Diabetes 46, 1087-1093 (1997).
Laubner, K., et al. Inhibition of preproinsulin gene expression by leptin induction of suppressor of cytokine signaling 3 in pancreatic beta-cells. Diabetes 54, 3410-3417 (2005).
Leibiger, B., et al. Insulin-feedback via PI3K-C2alpha activated PKBalpha/Aktl is required for glucose-stimulated insulin secretion. FASEB journal: official publication of the Federation of American Societies for Experimental Biology 24, 1824-1837 (2010).
Kerouz, N.J., Horsch, D., Pons, S. & Kahn, C.R. Differential regulation of insulin receptor substrates-1 and -2 (IRS-1 and IRS-2) and phosphatidylinositol 3-kinase isoforms in liver and muscle of the obese diabetic (ob/ob) mouse. J Clin Invest 100, 3164-3172 (1997).
Zawalich, W.S., Tesz, G.J. & Zawalich, K.C. Inhibitors of phosphatidylinositol 3-kinase amplify insulin release from islets of lean but not obese mice. The Journal of endocrinology 174, 247-258 (2002).
Kitamura, T., et al. The forkhead transcription factor Foxo1 links insulin signaling to Pdx1 regulation of pancreatic beta cell growth. J Clin Invest 110, 1839-1847 (2002).
Kitamura, Y.I., et al. FoxO1 protects against pancreatic beta cell failure through NeuroD and MafA induction. Cell metabolism 2, 153-163 (2005).
Okamoto, H., et al. Role of the forkhead protein FoxO1 in beta cell compensation to insulin resistance. J Clin Invest 116, 775-782 (2006).
Kamagate, A., et al. FoxO1 mediates insulin-dependent regulation of hepatic VLDL production in mice. J Clin Invest 118, 2347-2364 (2008).
Chen, W.M., et al. Variations in the G6PC2/ABCB11 genomic region are associated with fasting glucose levels. J Clin Invest 118, 2620-2628 (2008).
Wiegman, C.H., et al. Hepatic VLDL production in ob/ob mice is not stimulated by massive de novo lipogenesis but is less sensitive to the suppressive effects of insulin. Diabetes 52, 1081-1089 (2003).
Sol, E.M., Sundsten, T. & Bergsten, P. Role of MAPK in apolipoprotein CIII-induced apoptosis in INS-1E cells. Lipids in health and disease 8, 3 (2009).
Waanders, L.F., et al. Quantitative proteomic analysis of single pancreatic islets. Proceedings of the National Academy of Sciences of the United States of America 106, 18902-18907 (2009).
Kohler, M., et al. One-step purification of functional human and rat pancreatic alpha cells. Integrative biology : quantitative biosciences from nano to macro 4, 209-219 (2012).
Kluth, O., et al. Dissociation of lipotoxicity and glucotoxicity in a mouse model of obesity associated diabetes: role of forkhead box O1 (FOXO1) in glucose-induced beta cell failure. Diabetologia 54, 605-616 (2011).
Kulkarni, R.N., et al. beta-cell-specific deletion of the Igf1 receptor leads to hyperinsulinemia and glucose intolerance but does not alter beta-cell mass. Nature genetics 31, 111-115 (2002).
Xuan, S., et al. Defective insulin secretion in pancreatic beta cells lacking type 1 IGF receptor. J Clin Invest 110, 1011-1019 (2002).
Hribal, M.L., Nakae, J., Kitamura, T., Shutter, J.R. & Accili, D. Regulation of insulin-like growth factor-dependent myoblast differentiation by Foxo forkhead transcription factors. The Journal of cell biology 162, 535-541 (2003).
Kubota, N., et al. Disruption of insulin receptor substrate 2 causes type 2 diabetes because of liver insulin resistance and lack of compensatory beta-cell hyperplasia. Diabetes 49, 1880-1889 (2000).
Leibiger, I.B., Leibiger, B. & Berggren, P.O. Insulin signaling in the pancreatic beta-cell. Annual review of nutrition 28, 233-251 (2008).
Sakuraba, H., et al. Reduced beta-cell mass and expression of oxidative stress-related DNA damage in the islet of Japanese Type II diabetic patients. Diabetologia 45, 85-96 (2002).
Butler, A.E., et al. Beta-cell deficit and increased beta-cell apoptosis in humans with type 2 diabetes. Diabetes 52, 102-110 (2003).
Donath, M.Y., et al. Mechanisms of beta-cell death in type 2 diabetes. Diabetes 54 Suppl 2, S108-113 (2005).
Martinez, S.C., Cras-Meneur, C., Bernal-Mizrachi, E. & Permutt, M.A. Glucose regulates Foxo1 through insulin receptor signaling in the pancreatic islet beta-cell. Diabetes 55, 1581-1591 (2006).
Chan, D.C., Nguyen, M.N., Watts, G.F. & Barrett, P.H. Plasma apolipoprotein C-III transport in centrally obese men: associations with very low-density lipoprotein apolipoprotein B and high-density lipoprotein apolipoprotein A-I metabolism. The Journal of clinical endocrinology and metabolism 93, 557-564 (2008).
Joven, J., et al. Concentrations of lipids and apolipoproteins in patients with clinically well-controlled insulin-dependent and non-insulin-dependent diabetes. Clin Chem 35, 813-816 (1989).
al Muhtaseb, N., al Yousuf, A. & Bajaj, J.S. Apolipoprotein A-I, A-II, B, C-II, and C-III in children with insulin-dependent diabetes mellitus. Pediatrics 89, 936-941 (1992).
Stewart, M.W., Laker, M.F. & Alberti, K.G. The contribution of lipids to coronary heart disease in diabetes mellitus. J Intern Med Suppl 736, 41-46 (1994).
Hiukka, A., et al. Alterations of lipids and apolipoprotein CIII in very low density lipoprotein subspecies in type 2 diabetes. Diabetologia 48, 1207-1215 (2005).
Sundsten, T., Zethelius, B., Berne, C. & Bergsten, P. Plasma proteome changes in subjects with Type 2 diabetes mellitus with a low or high early insulin response. Clinical science 114, 499-507 (2008).
Hokanson, J.E., et al. Susceptibility to type 1 diabetes is associated with ApoCIII gene haplotypes. Diabetes 55, 834-838 (2006).
Pollin, T.I., et al. A null mutation in human APOC3 confers a favorable plasma lipid profile and apparent cardioprotection. Science 322, 1702-1705 (2008).
Atzmon, G., et al. Lipoprotein genotype and conserved pathway for exceptional longevity in humans. PLoS biology 4, e113 (2006).
Duivenvoorden, I., et al. Apolipoprotein C3 deficiency results in diet-induced obesity and aggravated insulin resistance in mice. Diabetes 54, 664-671 (2005).
Lee, H.Y., et al. Apolipoprotein CIII overexpressing mice are predisposed to diet-induced hepatic steatosis and hepatic insulin resistance. Hepatology 54, 1650-1660 (2011).
Kawakami, A., et al. Apolipoprotein CIII induces expression of vascular cell adhesion molecule-1 in vascular endothelial cells and increases adhesion of monocytic cells. Circulation 114, 681-687 (2006).
Hiukka, A., et al. ApoCIII-enriched LDL in type 2 diabetes displays altered lipid composition, increased susceptibility for sphingomyelinase, and increased binding to biglycan. Diabetes 58, 2018-2026 (2009).

## Claims

1. Composition comprising a compound of formula A-B, wherein A is a pancreatic βcell targeting moiety, and B is an inhibitor of expression and/or activity of Apolipoprotein CIII (apoCIII), and/or of β1 integrin for use in treating or limiting development of diabetes in a subject.

2. The composition for use of claim 1, wherein the pancreatic β cell specific targeting moiety comprises a moiety that selectively binds a protein selected from the group consisting of DiGeorge syndrome critical region gene 2 (DGCR2), golgi brefeldin A resistant guanine nucleotide exchange factor 1(GBF1), orphan G protein-coupled receptor GPR44(GPR44), SerpinB10 (PI-10), FXYD domain containing ion transport regulator 2 (FXYD2), Tetraspanin-7 (TSPAN7), gap junction protein, delta 2, 36kDa (GJD2), solute carrier family 18 (vesicular monoamine), member 2 (SLC18A2), prokineticin receptor 1 (PROKR1), glutamate receptor, metabotropic 5 (GRM5), neuropeptide Y receptor Y2 (NPY2R), glucagon-like peptide 1 receptor (GLP1R), and transmembrane protein 27 (TMEM27).

3. The composition for use of claim 1 or 2, wherein the pancreatic β cell specific targeting moiety comprises an antibody or an aptamer.

4. The composition for use of claim 1, wherein the pancreatic β cell specific targeting moiety comprises a moiety selected from the group consisting of glucagon-like peptide-1 (GLP-1), glucagon-like peptide-2 (GLP-2), peptide YY (PYY), neuropeptide Y (NPY), pancreatic peptide (PP), exendin-4, naphthylalanine and naphthylalanine derivatives.

5. The composition for use of any one of claims 1-4, wherein the inhibitor comprises an apoCIII inhibitor.

6. The composition for use of claim 5, wherein the apoCIII inhibitor is selected from the group consisting of anti-apoCIII antibody, anti-apoCIII aptamer, apoCIII small interfering RNA, apoCIII small internally segmented interfering RNA, apoCIII short hairpin RNA, apoCIII microRNA, and apoCIII antisense oligonucleotides.

7. The composition for use of any one of claims 1-6, wherein the inhibitor comprises a β1 integrin inhibitor, such as a β1 integrin inhibitor selected from the group consisting of anti-β1 integrin antibody, anti-β1 integrin aptamer, β1 integrin small interfering RNA, β1 integrin small internally segmented interfering RNA, β1 integrin short hairpin RNA, β1 integrin microRNA, and β1 integrin antisense oligonucleotides.

8. The composition for use of any one of claims 1- 7, wherein the subject overexpresses apoCIII relative to control.

9. The composition for use of any one of claims 1-8, wherein the subject has or is at risk of developing type 1 diabetes.

10. The composition for use of any one of claims 1- 8, wherein the subject has or is at risk of developing type 2 diabetes.

11. The composition for use of any one of claims 1- 8, wherein the subject has type 2 diabetes.

12. A composition comprising a compound of formula A-B, wherein A is a pancreatic β cell targeting moiety, and B is an inhibitor of Apolipoprotein CIII (apoCIII), or of β1 integrin.

13. The composition of claim 12, wherein the pancreatic β cell specific targeting moiety comprises a moiety that selectively binds a protein selected from the group consisting of DiGeorge syndrome critical region gene 2 (DGCR2), golgi brefeldin A resistant guanine nucleotide exchange factor 1 (GBF1), orphan G protein-coupled receptor GPR44(GPR44), SerpinB10 (PI-10), FXYD domain containing ion transport regulator 2 (FXYD2), Tetraspanin-7 (TSPAN7), gap junction protein, delta 2, 36kDa (GJD2), solute carrier family 18 (vesicular monoamine), member 2 (SLC18A2), prokineticin receptor 1 (PROKR1), glutamate receptor, metabotropic 5 (GRM5), neuropeptide Y receptor Y2 (NPY2R), glucagon-like peptide 1 receptor (GLP1R), and transmembrane protein 27 (TMEM27).

14. The composition of claim 12 or 13, wherein the pancreatic β cell specific targeting moiety comprises an antibody or an aptamer

15. The composition of any one of claims 12-14, wherein the pancreatic β cell specific targeting moiety comprises a moiety selected from the group consisting of glucagon-like peptide-1 (GLP-1), glucagon-like peptide-2 (GLP-2), peptide YY (PYY), neuropeptide Y (NPY), pancreatic peptide (PP), exendin-4, naphthylalanine and naphthylalanine derivatives

16. The composition of any one of claims 12-15, wherein the inhibitor comprises an ApoCIII inhibitor.

17. The composition of claim 16, wherein the inhibitor is selected from the group consisting of anti-apoCIII antibody, anti-apoCIII aptamer, apoCIII small interfering RNA, apoCIII small internally segmented interfering RNA, apoCIII short hairpin RNA, apoCIII microRNA, and apoCIII antisense oligonucleotides.

18. The composition of any one of claims 12-17, wherein the inhibitor comprises a β1 integrin inhibitor, such as an inhibitor selected from the group consisting of anti-β1 integrin antibody, anti-β1 integrin aptamer, β1 integrin small interfering RNA, β1 integrin small internally segmented interfering RNA, β1 integrin short hairpin RNA, β1 integrin microRNA, and β1 integrin antisense oligonucleotides.

19. The composition of any one of claims 12-18, wherein the composition comprises a compound of formula A-B-C, wherein C is a compound that permits cell entry of the composition, such as a cell penetrating peptide.

20. The composition of any one of claims 12-19, wherein the composition comprises a fusion protein.

21. The composition of any one of claims 12-19, wherein the composition comprises a recombinant nucleic acid.

22. An isolated nucleic acid encoding the fusion protein of claim 20 or the recombinant nucleic acid of claim 21, operatively linked to a promoter.

23. A recombinant expression vector comprising the isolated nucleic acid of claim 22.

24. An isolated recombinant host cell comprising the expression vector of claim 23.

## Patentansprüche

1. Zusammensetzung, die eine Verbindung der Formel A-B umfasst, wobei A eine Targeting-Gruppe für pankreatische β-Zellen ist und B ein Inhibitor der Expression und/oder der Aktivität von Apolipoprotein CIII (ApoCIII) und/oder von β1-Integrin ist, zur Behandlung oder Einschränkung der Entwicklung von Diabetes in einem Subjekt.

2. Zusammensetzung zur Verwendung nach Anspruch 1, wobei die für pankreatische-β-Zellen spezifische Targeting-Gruppe eine Gruppe umfasst, die selektiv ein Protein bindet, das aus der Gruppe ausgewählt ist, die aus der kritischen Region von Gen 2 des DiGeorge-Syndroms (DGCR2), dem Brefeldin-A-resistenten Golgi-Guanin-Nukleotid-Austauschfaktor 1 (GBF1), dem G-Protein-gekoppelten Orphan-Rezeptor GPR44 (GPR44), SerpinB10 (PI-10), dem FXYD-Domäne-haltigen Ionentransportregulator 2 (FXYD2), Tetraspanin-7 (TSPAN7), Gap-Junction-Protein, Delta 2, 36 kDa (GJD2), Solute-Carrier-Familie 18 (vesikuläres Monoamin) Mitglied 2 (SLC18A2), Prokineticin-Rezeptor 1 (PROKR1), Glutamatrezeptor, metabotrop 5 (GRM5), Neuropeptid-Y-Rezeptor Y2 (NPY2R), Glucagon-like-peptide-1-Rezeptor (GLP1R) und Transmembranprotein 27 (TMEM27) besteht.

3. Zusammensetzung zur Verwendung nach Anspruch 1 oder 2, wobei die für pankreatische-β-Zellen spezifische Targeting-Gruppe einen Antikörper oder ein Aptamer umfasst.

4. Zusammensetzung zur Verwendung nach Anspruch 1, wobei die für pankreatische-β-Zellen spezifische Targeting-Gruppe eine Gruppe umfasst, die aus der Gruppe ausgewählt ist, die aus Glucagon-like-peptide 1 (GLP-1), Glucagon-like-peptide 2 (GLP-2), Peptid YY (PYY), Neuropeptid Y (NPY), Pankreaspeptid (PP), Exendin-4, Naphthylalanin und Naphthylalaninderivaten besteht.

5. Zusammensetzung zur Verwendung nach einem der Ansprüche 1 bis 4, wobei der Inhibitor einen ApoCIII-Inhibitor umfasst.

6. Zusammensetzung zur Verwendung nach Anspruch 5, wobei der ApoCIII-Inhibitor aus der Gruppe ausgewählt ist, die aus Anti-ApoCIII-Antikörper, Anti-ApoCIII-Aptamer, kleine eingreifende (*small interfering*) ApoCIII-RNA, kleine, intern segmentierte, eingreifende ApoCIII-RNA, kurze ApoCIII-Haarnadel-RNA, ApoCIII-microRNA und ApoCIII-Antisense-Oligonukleotiden besteht.

7. Zusammensetzung zur Verwendung nach einem der Ansprüche 1 bis 6, wobei der Inhibitor einen β1-Integrin-Inhibitor umfasst, wie etwa einen β1-Integrin-Inhibitor, der aus der Gruppe ausgewählt ist, die aus Anti-β1-Integrin-Antikörper, Anti-β1-Integrin-Aptamer, kleine eingreifende (*small interfering*) β1-Integrin-RNA, kleine, intern segmentierte, eingreifende β1-Integrin-RNA, kurze β1-Integrin-Haarnadel-RNA, β1-Integrin-microRNA und β1-Integrin-Antisense-Oligonukleotiden besteht.

8. Zusammensetzung zur Verwendung nach einem der Ansprüche 1 bis 7, wobei das Subjekt ApoCIII relativ zur Kontrolle überexprimiert.

9. Zusammensetzung zur Verwendung nach einem der Ansprüche 1 bis 8, wobei das Subjekt Typ-1-Diabetes hat oder ein Risiko für die Entwicklung von Type-1-Diabetes aufweist.

10. Zusammensetzung zur Verwendung nach einem der Ansprüche 1 bis 8, wobei das Subjekt Typ-2-Diabetes hat oder ein Risiko für die Entwicklung von Typ-2-Diabetes aufweist.

11. Zusammensetzung zur Verwendung nach einem der Ansprüche 1 bis 8, wobei das Subjekt Typ-2-Diabetes hat.

12. Zusammensetzung, die eine Verbindung der Formel A-B umfasst, wobei A eine Targeting-Gruppe für pankreatische β-Zellen ist und B ein Inhibitor von Apolipoprotein CIII (ApoCIII) oder von β1-Integrin ist.

13. Zusammensetzung nach Anspruch 12, wobei die für pankreatische-β-Zellen spezifische Targeting-Gruppe eine Gruppe umfasst, die selektiv ein Protein bindet, das aus der Gruppe ausgewählt ist, die aus der kritischen Region von Gen 2 des DiGeorge-Syndroms (DGCR2), dem Brefeldin-A-resistenten Golgi-Guanin-Nukleotid-Austauschfaktor 1 (GBF1), dem G-Protein-gekoppelten Orphan-Rezeptor GPR44 (GPR44), SerpinB10 (PI-10), dem FXYD-Domäne-haltigen Ionentransportregulator 2 (FXYD2), Tetraspanin-7 (TSPAN7), Gap-Junction-Protein, Delta 2, 36 kDa (GJD2), Solute-Carrier-Familie 18 (vesikuläres Monoamin) Mitglied 2 (SLC18A2), Prokineticin-Rezeptor 1 (PROKR1), Glutamatrezeptor, metabotrop 5 (GRM5), Neuropeptid-Y-Rezeptor Y2 (NPY2R), Glucagon-like-peptide-1-Rezeptor (GLP1R) und Transmembranprotein 27 (TMEM27) besteht.

14. Zusammensetzung nach Anspruch 12 oder 13, wobei die für pankreatische-β-Zellen spezifische Targeting-Gruppe einen Antikörper oder ein Aptamer umfasst.

15. Zusammensetzung nach einem der Ansprüche 12 bis 14, wobei die für pankreatische-β-Zellen spezifische Targeting-Gruppe eine Gruppe umfasst, die aus der Gruppe ausgewählt ist, die aus Glucagon-like-peptide 1 (GLP-1), Glucagon-like-peptide 2 (GLP-2), Peptid YY (PYY), Neuropeptid Y (NPY), Pankreaspeptid (PP), Exendin-4, Naphthylalanin und Naphthylalaninderivaten besteht.

16. Zusammensetzung nach einem der Ansprüche 12 bis 15, wobei der Inhibitor einen ApoCIII-Inhibitor umfasst.

17. Zusammensetzung nach Anspruch 16, wobei der Inhibitor aus der Gruppe ausgewählt ist, die aus Anti-ApoCIII-Antikörper, Anti-ApoCIII-Aptamer, kleine eingreifende (*small interfering*) ApoCIII-RNA, kleine, intern segmentierte, eingreifende ApoCIII-RNA, kurze ApoCIII-Haarnadel-RNA, ApoCIII-microRNA und ApoCIII-Antisense-Oligonukleotiden besteht.

18. Zusammensetzung nach einem der Ansprüche 12 bis 17, wobei der Inhibitor einen β1-Integrin-Inhibitor umfasst, wie etwa einen β1-Integrin-Inhibitor, der aus der Gruppe ausgewählt ist, die aus Anti-β1-Integrin-Antikörper, Anti-β1-Integrin-Aptamer, kleine eingreifende (*small interfering*) β1-Integrin-RNA, kleine, intern segmentierte, eingreifende β1-Integrin-RNA, kurze β1-Integrin-Haarnadel-RNA, β1-Integrin-microRNA und β1-Integrin-Antisense-Oligonukleotiden besteht.

19. Zusammensetzung nach einem der Ansprüche 12 bis 18, wobei die Zusammensetzung eine Verbindung der Formel A-B-C umfasst, wobei C eine Verbindung ist, die den Eintritt der Zusammensetzung in die Zelle erlaubt, wie etwa ein zellpenetrierendes Peptid.

20. Zusammensetzung nach einem der Ansprüche 12 bis 19, wobei die Zusammensetzung ein Fusionsprotein umfasst.

21. Zusammensetzung nach einem der Ansprüche 12 bis 19, wobei die Zusammensetzung eine rekombinante Nukleinsäure umfasst.

22. Isolierte Nukleinsäure, die das Fusionsprotein nach Anspruch 20 oder die rekombinante Nukleinsäure nach Anspruch 21 kodiert, operativ verknüpft mit einem Promotor.

23. Rekombinanter Expressionsvektor, der die isolierte Nukleinsäure nach Anspruch 22 umfasst.

24. Isolierte rekombinante Wirtszelle, umfassend den Expressionsvektor nach Anspruch 23.

## Revendications

1. Composition comprenant un composé de formule A-B, dans laquelle A est une entité ciblant les cellules β pancréatiques, et B est un inhibiteur d'expression et/ou d'activité de l'Apolipoprotéine CIII (apoCIII), et/ou de l'intégrine β1 pour utilisation dans le traitement ou la limitation de l'évolution du diabète chez un sujet.

2. Composition pour utilisation selon la revendication 1, dans laquelle l'entité ciblant spécifiquement les cellules β pancréatiques comprend une entité qui se lie sélectivement une protéine choisie dans le groupe consistant en le gène 2 de la région critique pour le syndrome de DiGeorge (DGCR2), le facteur 1 d'échange de nucléotides de guanine résistant à la bréfeldine A de Golgi (GBF1), le récepteur GPR44 couplé à la protéine G orpheline (GPR44), la SerpineB10 (PI-10), le domaine FXYD contenant le régulateur de transport d'ions 2 (FXYD2), la Tétraspanine-7 (TSPAN7), la protéine de jonction communicante, delta 2, 36kDa (GJD2), la famille 18 de support de soluté (monoamine vésiculaire), le membre 2 (SLC18A2), le récepteur 1 de prokinéticine (PROKR1), le récepteur de glutamate, le métabotropique 5 (GRM5), le récepteur Y2 du neuropeptide Y (NPY2R), le récepteur de peptide 1 analogue au glucagon (GLP1R), et la protéine 27 transmembranaire (TMEM27).

3. Composition pour utilisation selon la revendication 1 ou 2, dans laquelle l'entité de ciblage spécifique des cellules β pancréatiques comprend un anticorps ou un aptamère.

4. Composition pour utilisation selon la revendication 1, dans laquelle l'entité de ciblage spécifique des cellules β pancréatiques comprend une entité choisie dans le groupe consistant en peptide-1 analogue au glucagon (GLP-1), peptide-2 analogue au glucagon (GLP-2), peptide YY (PYY), neuropeptide Y (NPY), peptide pancréatique (PP), exendine-4, naphtylalanine et dérivés de naphtylalanine.

5. Composition pour utilisation selon l'une quelconque des revendications 1-4, dans laquelle l'inhibiteur comprend un inhibiteur d'apoCIII.

6. Composition pour utilisation selon la revendication 5, dans laquelle l'inhibiteur d'apoCIII est choisi dans le groupe consistant en anticorps anti-apoCIII, aptamère anti-apoCIII, petit ARN interférant avec apoCIII, petit ARN interférant avec apoCIII segmenté en interne, ARN d'apoCIII en épingle à cheveux courte, microARN d'apoCIII, et oligonucléotides antisens d'apoCIII.

7. Composition pour utilisation selon l'une quelconque des revendications 1-6, dans laquelle l'inhibiteur comprend un inhibiteur d'intégrine β1, tel qu'un inhibiteur d'intégrine β1 choisi dans le groupe consistant en anticorps anti-intégrine β1, aptamère anti-intégrine β1, petit ARN interférant avec l'intégrine β1, petit ARN interférant avec l'intégrine β1 segmenté en interne, ARN d'intégrine β1 en épingle à cheveux courte, microARN d'intégrine β1, et oligonucléotides antisens d'intégrine β1.

8. Composition pour utilisation selon l'une quelconque des revendications 1-7, dans laquelle le sujet surexprime apoCIII par rapport à un témoin.

9. Composition pour utilisation selon l'une quelconque des revendications 1-8, dans laquelle le sujet a ou court le risque de développer un diabète de type 1.

10. Composition pour utilisation selon l'une quelconque des revendications 1-8, dans laquelle le sujet a ou court le risque de développer un diabète de type 2.

11. Composition pour utilisation selon l'une quelconque des revendications 1-8, dans laquelle le sujet a un diabète de type 2.

12. Composition comprenant un composé de formule A-B, dans laquelle A est une entité de ciblage des cellules β pancréatiques, et B est un inhibiteur d'apolipoprotéineCIII (apoCIII), ou d'intégrine β1.

13. Composition selon la revendication 12, dans laquelle l'entité de ciblage spécifique des cellules β pancréatiques comprend une entité qui se lie sélectivement une protéine choisie dans le groupe consistant en le gène 2 de la région critique pour le syndrome de DiGeorge (DGCR2), le facteur 1 d'échange de nucléotides de guanine résistant à la bréfeldine A de Golgi (GBF1), le récepteur GPR44 couplé à la protéine G orpheline (GPR44), la SerpineB10 (PI-10), le domaine FXYD contenant le régulateur de transport d'ions 2 (FXYD2), la Tétraspanine-7 (TSPAN7), la protéine de jonction communicante, delta 2, 36kDa (GJD2), la famille 18 de support de soluté (monoamine vésiculaire), le membre 2 (SLC18A2) le récepteur 1 de prokinéticine (PROKR1), le récepteur de glutamate, le métabotropique 5 (GRM5), le récepteur Y2 du neuropeptide Y (NPY2R), le récepteur de peptide 1 analogue au glucagon (GLP1R), et la protéine 27 transmembranaire (TMEM27).

14. Composition selon la revendication 12 ou 13, dans laquelle l'entité de ciblage spécifique des cellules β pancréatiques comprend un anticorps ou un aptamère.

15. Composition selon l'une quelconque des revendications 12-14, dans laquelle l'entité de ciblage spécifique des cellules β pancréatiques comprend une entité choisie dans le groupe consistant en peptide-1 analogue au glucagon (GLP-1), peptide-2 analogue au glucagon (GLP-2), peptide YY (PYY), neuropeptide Y (NPY), peptide pancréatique (PP), exendine-4, naphtylalanine et dérivés de naphtylalanine.

16. Composition selon l'une quelconque des revendications 12-15, dans laquelle l'inhibiteur comprend un inhibiteur d'apoCIII.

17. Composition selon la revendication 16, dans laquelle l'inhibiteur est choisi dans le groupe consistant en anticorps anti-apoCIII, aptamère anti-apoCIII, petit ARN interférant avec apoCIII, petit ARN interférant avec apoCIII segmenté en interne, ARN d'apoCIII en épingle à cheveux courte, microARN d'apoCIII, et oligonucléotides antisens d'apoCIII.

18. Composition selon l'une quelconque des revendications 12-17, dans laquelle l'inhibiteur comprend un inhibiteur d'intégrine β1, tel qu'un inhibiteur d'intégrine β1 choisi dans le groupe consistant en anticorps anti-intégrine β1, aptamère anti-intégrine β1, petit ARN interférant avec l'intégrine β1, petit ARN interférant avec l'intégrine β1 segmenté en interne, ARN d'intégrine β1 en épingle à cheveux courte, microARN d'intégrine β1, et oligonucléotides antisens d'intégrine β1.

19. Composition selon l'une quelconque des revendications 12-18, où la composition comprend un composé de formule A-B-C, tandis que C est un composé qui permet l'entrée de la composition dans les cellules, tel qu'un peptide pénétrant dans les cellules.

20. Composition selon l'une quelconque des revendications 12-19, où la composition comprend une protéine de fusion.

21. Composition selon l'une quelconque des revendications 12-19, où la composition comprend un acide nucléique recombinant.

22. Acide nucléique isolé codant pour la protéine de fusion selon la revendication 20 ou l'acide nucléique recombinant selon la revendication 21, lié de manière opérationnelle à un promoteur.

23. Vecteur d'expression recombinant comprenant l'acide nucléique isolé selon la revendication 22.

24. Cellule hôte recombinante isolée comprenant le vecteur d'expression selon la revendication 23.


### SEQUENCE LISTING

<110> Biocrine AB
<120> Methods for treating and/or limiting development of diabetes
<130> 150-334 PCT
<150> 61/779508
   <151> 2013-03-13
<160> 76
<170> PatentIn version 3.5
<210> 1
   <211> 180
   <212> PRT
   <213> Artificial Sequence
<220>
   <223> Synthetic
<400> 1
   Met Lys Ser Ile Tyr Phe Val Ala Gly Leu Phe Val Met Leu Val Gln
   1               5                   10                  15
   Gly Ser Trp Gln Arg Ser Leu Gln Asp Thr Glu Glu Lys Ser Arg Ser
               20                  25                  30
   Phe Ser Ala Ser Gln Ala Asp Pro Leu Ser Asp Pro Asp Gln Met Asn
           35                  40                  45
   Glu Asp Lys Arg His Ser Gln Gly Thr Phe Thr Ser Asp Tyr Ser Lys
       50                  55                  60
   Tyr Leu Asp Ser Arg Arg Ala Gln Asp Phe Val Gln Trp Leu Met Asn
   65                  70                  75                  80
   Thr Lys Arg Asn Arg Asn Asn Ile Ala Lys Arg His Asp Glu Phe Glu
                   85                  90                  95
   Arg His Ala Glu Gly Thr Phe Thr Ser Asp Val Ser Ser Tyr Leu Glu
               100                 105                 110
   Gly Gln Ala Ala Lys Glu Phe Ile Ala Trp Leu Val Lys Gly Arg Gly
           115                 120                 125
   Arg Arg Asp Phe Pro Glu Glu Val Ala Ile Val Glu Glu Leu Gly Arg
       130                 135                 140
   Arg His Ala Asp Gly Ser Phe Ser Asp Glu Met Asn Thr Ile Leu Asp
   145                 150                 155                 160
   Asn Leu Ala Ala Arg Asp Phe Ile Asn Trp Leu Ile Gln Thr Lys Ile
                   165                 170                 175
   Thr Asp Arg Lys
               180
<210> 2
<400> 2
   000
<210> 3
   <211> 97
   <212> PRT
   <213> Artificial Sequence
<220>
   <223> Synthetic
<400> 3
   Met Val Phe Val Arg Arg Pro Trp Pro Ala Leu Thr Thr Val Leu Leu
   1               5                   10                  15
   Ala Leu Leu Val Cys Leu Gly Ala Leu Val Asp Ala Tyr Pro Ile Lys
               20                  25                  30
   Pro Glu Ala Pro Gly Glu Asp Ala Ser Pro Glu Glu Leu Asn Arg Tyr
           35                  40                  45
   Tyr Ala Ser Leu Arg His Tyr Leu Asn Leu Val Thr Arg Gln Arg Tyr
       50                  55                  60
   Gly Lys Arg Asp Gly Pro Asp Thr Leu Leu Ser Lys Thr Phe Phe Pro
   65                  70                  75                  80
   Asp Gly Glu Asp Arg Pro Val Arg Ser Arg Ser Glu Gly Pro Asp Leu
                   85                  90                  95
   Trp
<210> 4
   <211> 97
   <212> PRT
   <213> Artificial Sequence
<220>
   <223> Synthetic
<400> 4
   Met Leu Gly Asn Lys Arg Leu Gly Leu Ser Gly Leu Thr Leu Ala Leu
   1               5                   10                  15
   Ser Leu Leu Val Cys Leu Gly Ala Leu Ala Glu Ala Tyr Pro Ser Lys
               20                  25                  30
   Pro Asp Asn Pro Gly Glu Asp Ala Pro Ala Glu Asp Met Ala Arg Tyr
           35                  40                  45
   Tyr Ser Ala Leu Arg His Tyr Ile Asn Leu Ile Thr Arg Gln Arg Tyr
       50                  55                  60
   Gly Lys Arg Ser Ser Pro Glu Thr Leu Ile Ser Asp Leu Leu Met Arg
   65                  70                  75                  80
   Glu Ser Thr Glu Asn Val Pro Arg Thr Arg Leu Glu Asp Pro Ala Met
                   85                  90                  95
   Trp
<210> 5
   <211> 95
   <212> PRT
   <213> Artificial Sequence
<220>
   <223> Synthetic
<400> 5
   Met Ala Ala Ala Arg Leu Cys Leu Ser Leu Leu Leu Leu Ser Thr Cys
   1               5                   10                  15
   Val Ala Leu Leu Leu Gln Pro Leu Leu Gly Ala Gln Gly Ala Pro Leu
               20                  25                  30
   Glu Pro Val Tyr Pro Gly Asp Asn Ala Thr Pro Glu Gln Met Ala Gln
           35                  40                  45
   Tyr Ala Ala Asp Leu Arg Arg Tyr Ile Asn Met Leu Thr Arg Pro Arg
       50                  55                  60
   Tyr Gly Lys Arg His Lys Glu Asp Thr Leu Ala Phe Ser Glu Trp Gly
   65                  70                  75                  80
   Ser Pro His Ala Ala Val Pro Arg Glu Leu Ser Pro Leu Asp Leu
                   85                  90                  95
<210> 6
   <211> 87
   <212> PRT
   <213> Artificial Sequence
<220>
   <223> Synthetic
<400> 6
   Met Lys Ile Ile Leu Trp Leu Cys Val Phe Gly Leu Phe Leu Ala Thr
   1               5                   10                  15
   Leu Phe Pro Ile Ser Trp Gln Met Pro Val Glu Ser Gly Leu Ser Ser
               20                  25                  30
   Glu Asp Ser Ala Ser Ser Glu Ser Phe Ala Ser Lys Ile Lys Arg His
           35                  40                  45
   Gly Glu Gly Thr Phe Thr Ser Asp Leu Ser Lys Gln Met Glu Glu Glu
       50                  55                  60
   Ala Val Arg Leu Phe Ile Glu Trp Leu Lys Asn Gly Gly Pro Ser Ser
   65                  70                  75                  80
   Gly Ala Pro Pro Pro Ser Gly
                   85
<210> 7
   <211> 550
   <212> PRT
   <213> Artificial Sequence
<220>
   <223> Synthetic
<400> 7
   Met Val Pro Lys Ala Asp Ser Gly Ala Phe Leu Leu Leu Phe Leu Leu
   1               5                   10                  15
   Val Leu Thr Val Thr Glu Pro Leu Arg Pro Glu Leu Arg Cys Asn Pro
               20                  25                  30
   Gly Gln Phe Ala Cys Arg Ser Gly Thr Ile Gln Cys Ile Pro Leu Pro
           35                  40                  45
   Trp Gln Cys Asp Gly Trp Ala Thr Cys Glu Asp Glu Ser Asp Glu Ala
       50                  55                  60
   Asn Cys Pro Glu Val Thr Gly Glu Val Arg Pro His His Gly Lys Glu
   65                  70                  75                  80
   Ala Val Asp Pro Arg Gln Gly Arg Ala Arg Gly Gly Asp Pro Ser His
                   85                  90                  95
   Phe His Ala Val Asn Val Ala Gln Pro Val Arg Phe Ser Ser Phe Leu
               100                 105                 110
   Gly Lys Cys Pro Thr Gly Trp His His Tyr Glu Gly Thr Ala Ser Cys
           115                 120                 125
   Tyr Arg Val Tyr Leu Ser Gly Glu Asn Tyr Trp Asp Ala Ala Gln Thr
       130                 135                 140
   Cys Gln Arg Leu Asn Gly Ser Leu Ala Thr Phe Ser Thr Asp Gln Glu
   145                 150                 155                 160
   Leu Arg Phe Val Leu Ala Gln Glu Trp Asp Gln Pro Glu Arg Ser Phe
                   165                 170                 175
   Gly Trp Lys Asp Gln Arg Lys Leu Trp Val Gly Tyr Gln Tyr Val Ile
               180                 185                 190
   Thr Gly Arg Asn Arg Ser Leu Glu Gly Arg Trp Glu Val Ala Phe Lys
           195                 200                 205
   Gly Ser Ser Glu Val Phe Leu Pro Pro Asp Pro Ile Phe Ala Ser Ala
       210                 215                 220
   Met Ser Glu Asn Asp Asn Val Phe Cys Ala Gln Leu Gln Cys Phe His
   225                 230                 235                 240
   Phe Pro Thr Leu Arg His His Asp Leu His Ser Trp His Ala Glu Ser
                   245                 250                 255
   Cys Tyr Glu Lys Ser Ser Phe Leu Cys Lys Arg Ser Gln Thr Cys Val
               260                 265                 270
   Asp Ile Lys Asp Asn Val Val Asp Glu Gly Phe Tyr Phe Thr Pro Lys
           275                 280                 285
   Gly Asp Asp Pro Cys Leu Ser Cys Thr Cys His Gly Gly Glu Pro Glu
       290                 295                 300
   Met Cys Val Ala Ala Leu Cys Glu Arg Pro Gln Gly Cys Gln Gln Tyr
   305                 310                 315                 320
   Arg Lys Asp Pro Lys Glu Cys Cys Lys Phe Met Cys Leu Asp Pro Asp
                   325                 330                 335
   Gly Asn Ser Leu Phe Asp Ser Met Ala Ser Gly Met Arg Leu Val Val
               340                 345                 350
   Ser Cys Ile Ser Ser Phe Leu Ile Leu Ser Leu Leu Leu Phe Met Val
           355                 360                 365
   His Arg Leu Arg Gln Arg Arg Arg Glu Arg Ile Glu Ser Leu Ile Gly
       370                 375                 380
   Ala Asn Leu His His Phe Asn Leu Gly Arg Arg Ile Pro Gly Phe Asp
   385                 390                 395                 400
   Tyr Gly Pro Asp Gly Phe Gly Thr Gly Leu Thr Pro Leu His Leu Ser
                   405                 410                 415
   Asp Asp Gly Glu Gly Gly Thr Phe His Phe His Asp Pro Pro Pro Pro
               420                 425                 430
   Tyr Thr Ala Tyr Lys Tyr Pro Asp Ile Gly Gln Pro Asp Asp Pro Pro
           435                 440                 445
   Pro Pro Tyr Glu Ala Ser Ile His Pro Asp Ser Val Phe Tyr Asp Pro
       450                 455                 460
   Ala Asp Asp Asp Ala Phe Glu Pro Val Glu Val Ser Leu Pro Ala Pro
   465                 470                 475                 480
   Gly Asp Gly Gly Ser Glu Gly Ala Leu Leu Arg Arg Leu Glu Gln Pro
                   485                 490                 495
   Leu Pro Thr Ala Gly Ala Ser Leu Ala Asp Leu Glu Asp Ser Ala Asp
               500                 505                 510
   Ser Ser Ser Ala Leu Leu Val Pro Pro Asp Pro Ala Gln Ser Gly Ser
           515                 520                 525
   Thr Pro Ala Ala Glu Ala Leu Pro Gly Gly Gly Arg His Ser Arg Ser
       530                 535                 540
   Ser Leu Asn Thr Val Val
   545                 550
<210> 8
   <211> 1859
   <212> PRT
   <213> Artificial Sequence
<220>
   <223> Synthetic
<400> 8
   Met Val Asp Lys Asn Ile Tyr Ile Ile Gln Gly Glu Ile Asn Ile Val
   1               5                   10                  15
   Val Gly Ala Ile Lys Arg Asn Ala Arg Trp Ser Thr His Thr Pro Leu
               20                  25                  30
   Asp Glu Glu Arg Asp Pro Leu Leu His Ser Phe Gly His Leu Lys Glu
           35                  40                  45
   Val Leu Asn Ser Ile Thr Glu Leu Ser Glu Ile Glu Pro Asn Val Phe
       50                  55                  60
   Leu Arg Pro Phe Leu Glu Val Ile Arg Ser Glu Asp Thr Thr Gly Pro
   65                  70                  75                  80
   Ile Thr Gly Leu Ala Leu Thr Ser Val Asn Lys Phe Leu Ser Tyr Ala
                   85                  90                  95
   Leu Ile Asp Pro Thr His Glu Gly Thr Ala Glu Gly Met Glu Asn Met
               100                 105                 110
   Ala Asp Ala Val Thr His Ala Arg Phe Val Gly Thr Asp Pro Ala Ser
           115                 120                 125
   Asp Glu Val Val Leu Met Lys Ile Leu Gln Val Leu Arg Thr Leu Leu
       130                 135                 140
   Leu Thr Pro Val Gly Ala His Leu Thr Asn Glu Ser Val Cys Glu Ile
   145                 150                 155                 160
   Met Gln Ser Cys Phe Arg Ile Cys Phe Glu Met Arg Leu Ser Glu Leu
                   165                 170                 175
   Leu Arg Lys Ser Ala Glu His Thr Leu Val Asp Met Val Gln Leu Leu
               180                 185                 190
   Phe Thr Arg Leu Pro Gln Phe Lys Glu Glu Pro Lys Asn Tyr Val Gly
           195                 200                 205
   Thr Asn Met Lys Lys Leu Lys Met Arg Ala Gly Gly Met Ser Asp Ser
       210                 215                 220
   Ser Lys Trp Lys Lys Gln Lys Arg Ser Pro Arg Pro Pro Arg His Met
   225                 230                 235                 240
   Thr Lys Val Thr Pro Gly Ser Glu Leu Pro Thr Pro Asn Gly Thr Thr
   245 250 255
   Leu Ser Ser Asn Leu Thr Gly Gly Met Pro Phe Ile Asp Val Pro Thr
   260 265 270
   Pro Ile Ser Ser Ala Ser Ser Glu Ala Ala Ser Ala Val Val Ser Pro
   275 280 285
   Ser Thr Asp Ser Gly Leu Glu Phe Ser Ser Gln Thr Thr Ser Lys Glu
   290 295 300
   Asp Leu Thr Asp Leu Glu Gln Pro Gly Ser Pro Gly Tyr Ser Thr Ala
   305 310 315 320
   Thr Glu Pro Gly Ser Ser Glu Leu Gly Val Pro Glu Gln Pro Asp Leu
   325 330 335
   Gln Glu Gly Thr His Val Glu Lys Ser Gln Ser Ala Ser Val Glu Ser
   340 345 350
   Ile Pro Glu Val Leu Glu Glu Cys Thr Ser Pro Ala Asp His Ser Asp
   355 360 365
   Ser Ala Ser Val His Asp Met Asp Tyr Val Asn Pro Arg Gly Val Arg
   370 375 380
   Phe Thr Gln Ser Ser Gln Lys Glu Gly Thr Ala Leu Val Pro Tyr Gly
   385 390 395 400
   Leu Pro Cys Ile Arg Glu Leu Phe Arg Phe Leu Ile Ser Leu Thr Asn
   405 410 415
   Pro His Asp Arg His Asn Ser Glu Val Met Ile His Met Gly Leu His
   420 425 430
   Leu Leu Thr Val Ala Leu Glu Ser Ala Pro Val Ala Gln Cys Gln Thr
   435 440 445
   Leu Leu Gly Leu Ile Lys Asp Glu Met Cys Arg His Leu Phe Gln Leu
   450 455 460
   Leu Ser Ile Glu Arg Leu Asn Leu Tyr Ala Ala Ser Leu Arg Val Cys
   465 470 475 480
   Phe Leu Leu Phe Glu Ser Met Arg Glu His Leu Lys Phe Gln Met Glu
   485 490 495
   Met Tyr Ile Lys Lys Leu Met Glu Ile Ile Thr Val Glu Asn Pro Lys
               500                 505                 510
   Met Pro Tyr Glu Met Lys Glu Met Ala Leu Glu Ala Ile Val Gln Leu
           515                 520                 525
   Trp Arg Ile Pro Ser Phe Val Thr Glu Leu Tyr Ile Asn Tyr Asp Cys
       530                 535                 540
   Asp Tyr Tyr Cys Ser Asn Leu Phe Glu Glu Leu Thr Lys Leu Leu Ser
   545                 550                 555                 560
   Lys Asn Ala Phe Pro Val Ser Gly Gln Leu Tyr Thr Thr His Leu Leu
                   565                 570                 575
   Ser Leu Asp Ala Leu Leu Thr Val Ile Asp Ser Thr Glu Ala His Cys
               580                 585                 590
   Gln Ala Lys Val Leu Asn Ser Leu Thr Gln Gln Glu Lys Lys Glu Thr
           595                 600                 605
   Ala Arg Pro Ser Cys Glu Ile Val Asp Gly Thr Arg Glu Ala Ser Asn
       610                 615                 620
   Thr Glu Arg Thr Ala Ser Asp Gly Lys Ala Val Gly Met Ala Ser Asp
   625                 630                 635                 640
   Ile Pro Gly Leu His Leu Pro Gly Gly Gly Arg Leu Pro Pro Glu His
                   645                 650                 655
   Gly Lys Ser Gly Cys Ser Asp Leu Glu Glu Ala Val Asp Ser Gly Ala
               660                 665                 670
   Asp Lys Lys Phe Ala Arg Lys Pro Pro Arg Phe Ser Cys Leu Leu Pro
           675                 680                 685
   Asp Pro Arg Glu Leu Ile Glu Ile Lys Asn Lys Lys Lys Leu Leu Ile
       690                 695                 700
   Thr Gly Thr Glu Gln Phe Asn Gln Lys Pro Lys Lys Gly Ile Gln Phe
   705                 710                 715                 720
   Leu Gln Glu Lys Gly Leu Leu Thr Ile Pro Met Asp Asn Thr Glu Val
                   725                 730                 735
   Ala Gln Trp Leu Arg Glu Asn Pro Arg Leu Asp Lys Lys Met Ile Gly
   740 745 750
   Glu Phe Val Ser Asp Arg Lys Asn Ile Asp Leu Leu Glu Ser Phe Val
   755 760 765
   Ser Thr Phe Ser Phe Gln Gly Leu Arg Leu Asp Glu Ala Leu Arg Leu
   770 775 780
   Tyr Leu Glu Ala Phe Arg Leu Pro Gly Glu Ala Pro Val Ile Gln Arg
   785 790 795 800
   Leu Leu Glu Ala Phe Thr Glu Arg Trp Met Asn Cys Asn Gly Ser Pro
   805 810 815
   Phe Ala Asn Ser Asp Ala Cys Phe Ser Leu Ala Tyr Ala Val Ile Met
   820 825 830
   Leu Asn Thr Asp Gln His Asn His Asn Val Arg Lys Gln Asn Ala Pro
   835 840 845
   Met Thr Leu Glu Glu Phe Arg Lys Asn Leu Lys Gly Val Asn Gly Gly
   850 855 860
   Lys Asp Phe Glu Gln Asp Ile Leu Glu Asp Met Tyr His Ala Ile Lys
   865 870 875 880
   Asn Glu Glu Ile Val Met Pro Glu Glu Gln Thr Gly Leu Val Arg Glu
   885 890 895
   Asn Tyr Val Trp Asn Val Leu Leu His Arg Gly Ala Thr Pro Glu Gly
   900 905 910
   Ile Phe Leu Arg Val Pro Thr Ala Ser Tyr Asp Leu Asp Leu Phe Thr
   915 920 925
   Met Thr Trp Gly Pro Thr Ile Ala Ala Leu Ser Tyr Val Phe Asp Lys
   930 935 940
   Ser Leu Glu Glu Thr Ile Ile Gln Lys Ala Ile Ser Gly Phe Arg Lys
   945 950 955 960
   Cys Ala Met Ile Ser Ala His Tyr Gly Leu Ser Asp Val Phe Asp Asn
   965 970 975
   Leu Ile Ile Ser Leu Cys Lys Phe Thr Ala Leu Ser Ser Glu Ser Ile
   980 985 990
   Glu Asn Leu Pro Ser Val Phe Gly  Ser Asn Pro Lys Ala  His Ile Ala
           995                 1000                 1005
   Ala Lys  Thr Val Phe His Leu  Ala His Arg His Gly  Asp Ile Leu
       1010                 1015                 1020
   Arg Glu  Gly Trp Lys Asn Ile  Met Glu Ala Met Leu  Gln Leu Phe
       1025                 1030                 1035
   Arg Ala  Gln Leu Leu Pro Lys  Ala Met Ile Glu Val  Glu Asp Phe
       1040                 1045                 1050
   Val Asp  Pro Asn Gly Lys Ile  Ser Leu Gln Arg Glu  Glu Thr Pro
       1055                 1060                 1065
   Ser Asn  Arg Gly Glu Ser Thr  Val Leu Ser Phe Val  Ser Trp Leu
       1070                 1075                 1080
   Thr Leu  Ser Gly Pro Glu Gln  Ser Ser Val Arg Gly  Pro Ser Thr
       1085                 1090                 1095
   Glu Asn  Gln Glu Ala Lys Arg  Val Ala Leu Glu Cys  Ile Lys Gln
       1100                 1105                 1110
   Cys Asp  Pro Glu Lys Met Ile  Thr Glu Ser Lys Phe  Leu Gln Leu
       1115                 1120                 1125
   Glu Ser  Leu Gln Glu Leu Met  Lys Ala Leu Val Ser  Val Thr Pro
       1130                 1135                 1140
   Asp Glu  Glu Thr Tyr Asp Glu  Glu Asp Ala Ala Phe  Cys Leu Glu
       1145                 1150                 1155
   Met Leu  Leu Arg Ile Val Leu  Glu Asn Arg Asp Arg  Val Gly Cys
       1160                 1165                 1170
   Val Trp  Gln Thr Val Arg Asp  His Leu Tyr His Leu  Cys Val Gln
       1175                 1180                 1185
   Ala Gln  Asp Phe Cys Phe Leu  Val Glu Arg Ala Val  Val Gly Leu
       1190                 1195                 1200
   Leu Arg  Leu Ala Ile Arg Leu  Leu Arg Arg Glu Glu  Ile Ser Ala
       1205                 1210                 1215
   Gln Val  Leu Leu Ser Leu Arg  Ile Leu Leu Leu Met  Lys Pro Ser
       1220                 1225                 1230
   Val Leu  Ser Arg Val Ser His  Gln Val Ala Tyr Gly  Leu His Glu
       1235                 1240                 1245
   Leu Leu  Lys Thr Asn Ala Ala  Asn Ile His Ser Gly  Asp Asp Trp
       1250                 1255                 1260
   Ala Thr  Leu Phe Thr Leu Leu  Glu Cys Ile Gly Ser  Gly Val Lys
       1265                 1270                 1275
   Pro Pro  Ala Ala Leu Gln Ala  Thr Ala Arg Ala Asp  Ala Pro Asp
       1280                 1285                 1290
   Ala Gly  Ala Gln Ser Asp Ser  Glu Leu Pro Ser Tyr  His Gln Asn
       1295                 1300                 1305
   Asp Val  Ser Leu Asp Arg Gly  Tyr Thr Ser Asp Ser  Glu Val Tyr
       1310                 1315                 1320
   Thr Asp  His Gly Arg Pro Gly  Lys Ile His Arg Ser  Ala Thr Asp
       1325                 1330                 1335
   Ala Asp  Val Val Asn Ser Gly  Trp Leu Val Val Gly  Lys Asp Asp
       1340                 1345                 1350
   Val Asp  Asn Ser Lys Pro Gly  Pro Ser Arg Pro Gly  Pro Ser Pro
       1355                 1360                 1365
   Leu Ile  Asn Gln Tyr Ser Leu  Thr Val Gly Leu Asp  Leu Gly Pro
       1370                 1375                 1380
   His Asp  Thr Lys Ser Leu Leu  Lys Cys Val Glu Ser  Leu Ser Phe
       1385                 1390                 1395
   Ile Val  Arg Asp Ala Ala His  Ile Thr Pro Asp Asn  Phe Glu Leu
       1400                 1405                 1410
   Cys Val  Lys Thr Leu Arg Ile  Phe Val Glu Ala Ser  Leu Asn Gly
       1415                 1420                 1425
   Gly Cys  Lys Ser Gln Glu Lys  Arg Gly Lys Ser His  Lys Tyr Asp
       1430                 1435                 1440
   Ser Lys  Gly Asn Arg Phe Lys  Lys Lys Ser Lys Glu  Gly Ser Met
       1445                 1450                 1455
   Leu Arg  Arg Pro Arg Thr Ser  Ser Gln His Ala Ser  Arg Gly Gly
       1460                 1465                 1470
   Gln Ser  Asp Asp Asp Glu Asp  Glu Gly Val Pro Ala  Ser Tyr His
       1475                 1480                 1485
   Thr Val  Ser Leu Gln Val Ser  Gln Asp Leu Leu Asp  Leu Met His
       1490                 1495                 1500
   Thr Leu  His Thr Arg Ala Ala  Ser Ile Tyr Ser Ser  Trp Ala Glu
       1505                 1510                 1515
   Glu Gln  Arg His Leu Glu Thr  Gly Gly Gln Lys Ile  Glu Ala Asp
       1520                 1525                 1530
   Ser Arg  Thr Leu Trp Ala His  Cys Trp Cys Pro Leu  Leu Gln Gly
       1535                 1540                 1545
   Ile Ala  Cys Leu Cys Cys Asp  Ala Arg Arg Gln Val  Arg Met Gln
       1550                 1555                 1560
   Ala Leu  Thr Tyr Leu Gln Arg  Ala Leu Leu Val His  Asp Leu Gln
       1565                 1570                 1575
   Lys Leu  Asp Ala Leu Glu Trp  Glu Ser Cys Phe Asn  Lys Val Leu
       1580                 1585                 1590
   Phe Pro  Leu Leu Thr Lys Leu  Leu Glu Asn Ile Ser  Pro Ala Asp
       1595                 1600                 1605
   Val Gly  Gly Met Glu Glu Thr  Arg Met Arg Ala Ser  Thr Leu Leu
       1610                 1615                 1620
   Ser Lys  Val Phe Leu Gln His  Leu Ser Pro Leu Leu  Ser Leu Ser
       1625                 1630                 1635
   Thr Phe  Ala Ala Leu Trp Leu  Thr Ile Leu Asp Phe  Met Asp Lys
       1640                 1645                 1650
   Tyr Met  His Ala Gly Ser Ser  Asp Leu Leu Ser Glu  Ala Ile Pro
       1655                 1660                 1665
   Glu Ser  Leu Lys Asn Met Leu  Leu Val Met Asp Thr  Ala Glu Ile
       1670                 1675                 1680
   Phe His  Ser Ala Asp Ala Arg  Gly Gly Gly Pro Ser  Ala Leu Trp
       1685                 1690                 1695
   Glu Ile  Thr Trp Glu Arg Ile  Asp Cys Phe Leu Pro  His Leu Arg
   1700 1705 1710
   Asp Glu Leu Phe Lys Gln Thr Val Ile Gln Asp Pro Met Pro Met
   1715 1720 1725
   Glu Pro Gln Gly Gln Lys Pro Leu Ala Ser Ala His Leu Thr Ser
   1730 1735 1740
   Ala Ala Gly Asp Thr Arg Thr Pro Gly His Pro Pro Pro Pro Glu
   1745 1750 1755
   Ile Pro Ser Glu Leu Gly Ala Cys Asp Phe Glu Lys Pro Glu Ser
   1760 1765 1770
   Pro Arg Ala Ala Ser Ser Ser Ser Pro Gly Ser Pro Val Ala Ser
   1775 1780 1785
   Ser Pro Ser Arg Leu Ser Pro Thr Pro Asp Gly Pro Pro Pro Leu
   1790 1795 1800
   Ala Gln Pro Pro Leu Ile Leu Gln Pro Leu Ala Ser Pro Leu Gln
   1805 1810 1815
   Val Gly Val Pro Pro Met Thr Leu Pro Ile Ile Leu Asn Pro Ala
   1820 1825 1830
   Leu Ile Glu Ala Thr Ser Pro Val Pro Leu Leu Ala Thr Pro Arg
   1835 1840 1845
   Pro Thr Asp Pro Ile Pro Thr Ser Glu Val Asn
   1850 1855
<210> 9
   <211> 502
   <212> PRT
   <213> Artificial Sequence
<220>
   <223> Synthetic
<400> 9
<210> 10
   <211> 397
   <212> PRT
   <213> Artificial Sequence
<220>
   <223> Synthetic
<400> 10
   Met Asp Ser Leu Ala Thr Ser Ile Asn Gln Phe Ala Leu Glu Leu Ser
   1               5                   10                  15
   Lys Lys Leu Ala Glu Ser Ala Gln Gly Lys Asn Ile Phe Phe Ser Ser
               20                  25                  30
   Trp Ser Ile Ser Thr Ser Leu Thr Ile Val Tyr Leu Gly Ala Lys Gly
           35                  40                  45
   Thr Thr Ala Ala Gln Met Ala Gln Val Leu Gln Phe Asn Arg Asp Gln
       50                  55                  60
   Gly Val Lys Cys Asp Pro Glu Ser Glu Lys Lys Arg Lys Met Glu Phe
   65                  70                  75                  80
   Asn Leu Ser Asn Ser Glu Glu Ile His Ser Asp Phe Gln Thr Leu Ile
                   85                  90                  95
   Ser Glu Ile Leu Lys Pro Asn Asp Asp Tyr Leu Leu Lys Thr Ala Asn
               100                 105                 110
   Ala Ile Tyr Gly Glu Lys Thr Tyr Ala Phe His Asn Lys Tyr Leu Glu
           115                 120                 125
   Asp Met Lys Thr Tyr Phe Gly Ala Glu Pro Gln Pro Val Asn Phe Val
       130                 135                 140
   Glu Ala Ser Asp Gln Ile Arg Lys Asp Ile Asn Ser Trp Val Glu Arg
   145                 150                 155                 160
   Gln Thr Glu Gly Lys Ile Gln Asn Leu Leu Pro Asp Asp Ser Val Asp
                   165                 170                 175
   Ser Thr Thr Arg Met Ile Leu Val Asn Ala Leu Tyr Phe Lys Gly Ile
               180                 185                 190
   Trp Glu His Gln Phe Leu Val Gln Asn Thr Thr Glu Lys Pro Phe Arg
           195                 200                 205
   Ile Asn Glu Thr Thr Ser Lys Pro Val Gln Met Met Phe Met Lys Lys
       210                 215                 220
   Lys Leu His Ile Phe His Ile Glu Lys Pro Lys Ala Val Gly Leu Gln
   225                 230                 235                 240
   Leu Tyr Tyr Lys Ser Arg Asp Leu Ser Leu Leu Ile Leu Leu Pro Glu
   245 250 255
   Asp Ile Asn Gly Leu Glu Gln Leu Glu Lys Ala Ile Thr Tyr Glu Lys
   260 265 270
   Leu Asn Glu Trp Thr Ser Ala Asp Met Met Glu Leu Tyr Glu Val Gln
   275 280 285
   Leu His Leu Pro Lys Phe Lys Leu Glu Asp Ser Tyr Asp Leu Lys Ser
   290 295 300
   Thr Leu Ser Ser Met Gly Met Ser Asp Ala Phe Ser Gln Ser Lys Ala
   305 310 315 320
   Asp Phe Ser Gly Met Ser Ser Ala Arg Asn Leu Phe Leu Ser Asn Val
   325 330 335
   Phe His Lys Ala Phe Val Glu Ile Asn Glu Gln Gly Thr Glu Ala Ala
   340 345 350
   Ala Gly Ser Gly Ser Glu Ile Asp Ile Arg Ile Arg Val Pro Ser Ile
   355 360 365
   Glu Phe Asn Ala Asn His Pro Phe Leu Phe Phe Ile Arg His Asn Lys
   370 375 380
   Thr Asn Thr Ile Leu Phe Tyr Gly Arg Leu Cys Ser Pro
   385 390 395
<210> 11
   <211> 66
   <212> PRT
   <213> Artificial Sequence
<220>
   <223> Synthetic
<400> 11
<210> 12
   <211> 249
   <212> PRT
   <213> Artificial Sequence
<220>
   <223> Synthetic
<400> 12
   Met Ala Ser Arg Arg Met Glu Thr Lys Pro Val Ile Thr Cys Leu Lys
   1               5                   10                  15
   Thr Leu Leu Ile Ile Tyr Ser Phe Val Phe Trp Ile Thr Gly Val Ile
               20                  25                  30
   Leu Leu Ala Val Gly Val Trp Gly Lys Leu Thr Leu Gly Thr Tyr Ile
           35                  40                  45
   Ser Leu Ile Ala Glu Asn Ser Thr Asn Ala Pro Tyr Val Leu Ile Gly
       50                  55                  60
   Thr Gly Thr Thr Ile Val Val Phe Gly Leu Phe Gly Cys Phe Ala Thr
   65                  70                  75                  80
   Cys Arg Gly Ser Pro Trp Met Leu Lys Leu Tyr Ala Met Phe Leu Ser
                   85                  90                  95
   Leu Val Phe Leu Ala Glu Leu Val Ala Gly Ile Ser Gly Phe Val Phe
               100                 105                 110
   Arg His Glu Ile Lys Asp Thr Phe Leu Arg Thr Tyr Thr Asp Ala Met
           115                 120                 125
   Gln Thr Tyr Asn Gly Asn Asp Glu Arg Ser Arg Ala Val Asp His Val
       130                 135                 140
   Gln Arg Ser Leu Ser Cys Cys Gly Val Gln Asn Tyr Thr Asn Trp Ser
   145                 150                 155                 160
   Thr Ser Pro Tyr Phe Leu Glu His Gly Ile Pro Pro Ser Cys Cys Met
                   165                 170                 175
   Asn Glu Thr Asp Cys Asn Pro Gln Asp Leu His Asn Leu Thr Val Ala
               180                 185                 190
   Ala Thr Lys Val Asn Gln Lys Gly Cys Tyr Asp Leu Val Thr Ser Phe
           195                 200                 205
   Met Glu Thr Asn Met Gly Ile Ile Ala Gly Val Ala Phe Gly Ile Ala
       210                 215                 220
   Phe Ser Gln Leu Ile Gly Met Leu Leu Ala Cys Cys Leu Ser Arg Phe
   225                 230                 235                 240
   Ile Thr Ala Asn Gln Tyr Glu Met Val
                   245
<210> 13
   <211> 321
   <212> PRT
   <213> Artificial Sequence
<220>
   <223> Synthetic
<400> 13
   Met Gly Glu Trp Thr Ile Leu Glu Arg Leu Leu Glu Ala Ala Val Gln
   1               5                   10                  15
   Gln His Ser Thr Met Ile Gly Arg Ile Leu Leu Thr Val Val Val Ile
               20                  25                  30
   Phe Arg Ile Leu Ile Val Ala Ile Val Gly Glu Thr Val Tyr Asp Asp
           35                  40                  45
   Glu Gln Thr Met Phe Val Cys Asn Thr Leu Gln Pro Gly Cys Asn Gln
       50                  55                  60
   Ala Cys Tyr Asp Arg Ala Phe Pro Ile Ser His Ile Arg Tyr Trp Val
   65                  70                  75                  80
   Phe Gln Ile Ile Met Val Cys Thr Pro Ser Leu Cys Phe Ile Thr Tyr
                   85                  90                  95
   Ser Val His Gln Ser Ala Lys Gln Arg Glu Arg Arg Tyr Ser Thr Val
               100                 105                 110
   Phe Leu Ala Leu Asp Arg Asp Pro Pro Glu Ser Ile Gly Gly Pro Gly
           115                 120                 125
   Gly Thr Gly Gly Gly Gly Ser Gly Gly Gly Lys Arg Glu Asp Lys Lys
       130                 135                 140
   Leu Gln Asn Ala Ile Val Asn Gly Val Leu Gln Asn Thr Glu Asn Thr
   145                 150                 155                 160
   Ser Lys Glu Thr Glu Pro Asp Cys Leu Glu Val Lys Glu Leu Thr Pro
                   165                 170                 175
   His Pro Ser Gly Leu Arg Thr Ala Ser Lys Ser Lys Leu Arg Arg Gln
               180                 185                 190
   Glu Gly Ile Ser Arg Phe Tyr Ile Ile Gln Val Val Phe Arg Asn Ala
           195                 200                 205
   Leu Glu Ile Gly Phe Leu Val Gly Gln Tyr Phe Leu Tyr Gly Phe Ser
       210                 215                 220
   Val Pro Gly Leu Tyr Glu Cys Asn Arg Tyr Pro Cys Ile Lys Glu Val
   225                 230                 235                 240
   Glu Cys Tyr Val Ser Arg Pro Thr Glu Lys Thr Val Phe Leu Val Phe
                   245                 250                 255
   Met Phe Ala Val Ser Gly Ile Cys Val Val Leu Asn Leu Ala Glu Leu
               260                 265                 270
   Asn His Leu Gly Trp Arg Lys Ile Lys Leu Ala Val Arg Gly Ala Gln
           275                 280                 285
   Ala Lys Arg Lys Ser Ile Tyr Glu Ile Arg Asn Lys Asp Leu Pro Arg
       290                 295                 300
   Val Ser Val Pro Asn Phe Gly Arg Thr Gln Ser Ser Asp Ser Ala Tyr
   305                 310                 315                 320
   Val
<210> 14
   <211> 514
   <212> PRT
   <213> Artificial Sequence
<220>
   <223> Synthetic
<400> 14
<210> 15
   <211> 393
   <212> PRT
   <213> Artificial Sequence
<220>
   <223> Synthetic
<400> 15
   Met Glu Thr Thr Met Gly Phe Met Asp Asp Asn Ala Thr Asn Thr Ser
   1               5                   10                  15
   Thr Ser Phe Leu Ser Val Leu Asn Pro His Gly Ala His Ala Thr Ser
               20                  25                  30
   Phe Pro Phe Asn Phe Ser Tyr Ser Asp Tyr Asp Met Pro Leu Asp Glu
           35                  40                  45
   Asp Glu Asp Val Thr Asn Ser Arg Thr Phe Phe Ala Ala Lys Ile Val
       50                  55                  60
   Ile Gly Met Ala Leu Val Gly Ile Met Leu Val Cys Gly Ile Gly Asn
   65                  70                  75                  80
   Phe Ile Phe Ile Ala Ala Leu Val Arg Tyr Lys Lys Leu Arg Asn Leu
                   85                  90                  95
   Thr Asn Leu Leu Ile Ala Asn Leu Ala Ile Ser Asp Phe Leu Val Ala
               100                 105                 110
   Ile Val Cys Cys Pro Phe Glu Met Asp Tyr Tyr Val Val Arg Gln Leu
           115                 120                 125
   Ser Trp Glu His Gly His Val Leu Cys Thr Ser Val Asn Tyr Leu Arg
       130                 135                 140
   Thr Val Ser Leu Tyr Val Ser Thr Asn Ala Leu Leu Ala Ile Ala Ile
   145                 150                 155                 160
   Asp Arg Tyr Leu Ala Ile Val His Pro Leu Arg Pro Arg Met Lys Cys
                   165                 170                 175
   Gln Thr Ala Thr Gly Leu Ile Ala Leu Val Trp Thr Val Ser Ile Leu
               180                 185                 190
   Ile Ala Ile Pro Ser Ala Tyr Phe Thr Thr Glu Thr Val Leu Val Ile
           195                 200                 205
   Val Lys Ser Gln Glu Lys Ile Phe Cys Gly Gln Ile Trp Pro Val Asp
       210                 215                 220
   Gln Gln Leu Tyr Tyr Lys Ser Tyr Phe Leu Phe Ile Phe Gly Ile Glu
   225                 230                 235                 240
   Phe Val Gly Pro Val Val Thr Met Thr Leu Cys Tyr Ala Arg Ile Ser
                   245                 250                 255
   Arg Glu Leu Trp Phe Lys Ala Val Pro Gly Phe Gln Thr Glu Gln Ile
               260                 265                 270
   Arg Lys Arg Leu Arg Cys Arg Arg Lys Thr Val Leu Val Leu Met Cys
           275                 280                 285
   Ile Leu Thr Ala Tyr Val Leu Cys Trp Ala Pro Phe Tyr Gly Phe Thr
       290                 295                 300
   Ile Val Arg Asp Phe Phe Pro Thr Val Phe Val Lys Glu Lys His Tyr
   305                 310                 315                 320
   Leu Thr Ala Phe Tyr Ile Val Glu Cys Ile Ala Met Ser Asn Ser Met
                   325                 330                 335
   Ile Asn Thr Leu Cys Phe Val Thr Val Lys Asn Asp Thr Val Lys Tyr
               340                 345                 350
   Phe Lys Lys Ile Met Leu Leu His Trp Lys Ala Ser Tyr Asn Gly Gly
           355                 360                 365
   Lys Ser Ser Ala Asp Leu Asp Leu Lys Thr Ile Gly Met Pro Ala Thr
       370                 375                 380
   Glu Glu Val Asp Cys Ile Arg Leu Lys
   385                 390
<210> 16
   <211> 1212
   <212> PRT
   <213> Artificial Sequence
<220>
   <223> Synthetic
<400> 16
   Met Val Leu Leu Leu Ile Leu Ser Val Leu Leu Leu Lys Glu Asp Val
   1               5                   10                  15
   Arg Gly Ser Ala Gln Ser Ser Glu Arg Arg Val Val Ala His Met Pro
               20                  25                  30
   Gly Asp Ile Ile Ile Gly Ala Leu Phe Ser Val His His Gln Pro Thr
           35                  40                  45
   Val Asp Lys Val His Glu Arg Lys Cys Gly Ala Val Arg Glu Gln Tyr
       50                  55                  60
   Gly Ile Gln Arg Val Glu Ala Met Leu His Thr Leu Glu Arg Ile Asn
   65                  70                  75                  80
   Ser Asp Pro Thr Leu Leu Pro Asn Ile Thr Leu Gly Cys Glu Ile Arg
                   85                  90                  95
   Asp Ser Cys Trp His Ser Ala Val Ala Leu Glu Gln Ser Ile Glu Phe
               100                 105                 110
   Ile Arg Asp Ser Leu Ile Ser Ser Glu Glu Glu Glu Gly Leu Val Arg
           115                 120                 125
   Cys Val Asp Gly Ser Ser Ser Ser Phe Arg Ser Lys Lys Pro Ile Val
       130                 135                 140
   Gly Val Ile Gly Pro Gly Ser Ser Ser Val Ala Ile Gln Val Gln Asn
   145                 150                 155                 160
   Leu Leu Gln Leu Phe Asn Ile Pro Gln Ile Ala Tyr Ser Ala Thr Ser
                   165                 170                 175
   Met Asp Leu Ser Asp Lys Thr Leu Phe Lys Tyr Phe Met Arg Val Val
               180                 185                 190
   Pro Ser Asp Ala Gln Gln Ala Arg Ala Met Val Asp Ile Val Lys Arg
           195                 200                 205
   Tyr Asn Trp Thr Tyr Val Ser Ala Val His Thr Glu Gly Asn Tyr Gly
       210                 215                 220
   Glu Ser Gly Met Glu Ala Phe Lys Asp Met Ser Ala Lys Glu Gly Ile
   225                 230                 235                 240
   Cys Ile Ala His Ser Tyr Lys Ile Tyr Ser Asn Ala Gly Glu Gln Ser
                   245                 250                 255
   Phe Asp Lys Leu Leu Lys Lys Leu Thr Ser His Leu Pro Lys Ala Arg
               260                 265                 270
   Val Val Ala Cys Phe Cys Glu Gly Met Thr Val Arg Gly Leu Leu Met
           275                 280                 285
   Ala Met Arg Arg Leu Gly Leu Ala Gly Glu Phe Leu Leu Leu Gly Ser
       290                 295                 300
   Asp Gly Trp Ala Asp Arg Tyr Asp Val Thr Asp Gly Tyr Gln Arg Glu
   305                 310                 315                 320
   Ala Val Gly Gly Ile Thr Ile Lys Leu Gln Ser Pro Asp Val Lys Trp
                   325                 330                 335
   Phe Asp Asp Tyr Tyr Leu Lys Leu Arg Pro Glu Thr Asn His Arg Asn
               340                 345                 350
   Pro Trp Phe Gln Glu Phe Trp Gln His Arg Phe Gln Cys Arg Leu Glu
           355                 360                 365
   Gly Phe Pro Gln Glu Asn Ser Lys Tyr Asn Lys Thr Cys Asn Ser Ser
       370                 375                 380
   Leu Thr Leu Lys Thr His His Val Gln Asp Ser Lys Met Gly Phe Val
   385                 390                 395                 400
   Ile Asn Ala Ile Tyr Ser Met Ala Tyr Gly Leu His Asn Met Gln Met
                   405                 410                 415
   Ser Leu Cys Pro Gly Tyr Ala Gly Leu Cys Asp Ala Met Lys Pro Ile
               420                 425                 430
   Asp Gly Arg Lys Leu Leu Glu Ser Leu Met Lys Thr Asn Phe Thr Gly
           435                 440                 445
   Val Ser Gly Asp Thr Ile Leu Phe Asp Glu Asn Gly Asp Ser Pro Gly
       450                 455                 460
   Arg Tyr Glu Ile Met Asn Phe Lys Glu Met Gly Lys Asp Tyr Phe Asp
   465                 470                 475                 480
   Tyr Ile Asn Val Gly Ser Trp Asp Asn Gly Glu Leu Lys Met Asp Asp
                   485                 490                 495
   Asp Glu Val Trp Ser Lys Lys Ser Asn Ile Ile Arg Ser Val Cys Ser
               500                 505                 510
   Glu Pro Cys Glu Lys Gly Gln Ile Lys Val Ile Arg Lys Gly Glu Val
           515                 520                 525
   Ser Cys Cys Trp Thr Cys Thr Pro Cys Lys Glu Asn Glu Tyr Val Phe
       530                 535                 540
   Asp Glu Tyr Thr Cys Lys Ala Cys Gln Leu Gly Ser Trp Pro Thr Asp
   545                 550                 555                 560
   Asp Leu Thr Gly Cys Asp Leu Ile Pro Val Gln Tyr Leu Arg Trp Gly
                   565                 570                 575
   Asp Pro Glu Pro Ile Ala Ala Val Val Phe Ala Cys Leu Gly Leu Leu
               580                 585                 590
   Ala Thr Leu Phe Val Thr Val Val Phe Ile Ile Tyr Arg Asp Thr Pro
           595                 600                 605
   Val Val Lys Ser Ser Ser Arg Glu Leu Cys Tyr Ile Ile Leu Ala Gly
       610                 615                 620
   Ile Cys Leu Gly Tyr Leu Cys Thr Phe Cys Leu Ile Ala Lys Pro Lys
   625                 630                 635                 640
   Gln Ile Tyr Cys Tyr Leu Gln Arg Ile Gly Ile Gly Leu Ser Pro Ala
                   645                 650                 655
   Met Ser Tyr Ser Ala Leu Val Thr Lys Thr Asn Arg Ile Ala Arg Ile
               660                 665                 670
   Leu Ala Gly Ser Lys Lys Lys Ile Cys Thr Lys Lys Pro Arg Phe Met
           675                 680                 685
   Ser Ala Cys Ala Gln Leu Val Ile Ala Phe Ile Leu Ile Cys Ile Gln
       690                 695                 700
   Leu Gly Ile Ile Val Ala Leu Phe Ile Met Glu Pro Pro Asp Ile Met
   705                 710                 715                 720
   His Asp Tyr Pro Ser Ile Arg Glu Val Tyr Leu Ile Cys Asn Thr Thr
                   725                 730                 735
   Asn Leu Gly Val Val Thr Pro Leu Gly Tyr Asn Gly Leu Leu Ile Leu
               740                 745                 750
   Ser Cys Thr Phe Tyr Ala Phe Lys Thr Arg Asn Val Pro Ala Asn Phe
           755                 760                 765
   Asn Glu Ala Lys Tyr Ile Ala Phe Thr Met Tyr Thr Thr Cys Ile Ile
       770                 775                 780
   Trp Leu Ala Phe Val Pro Ile Tyr Phe Gly Ser Asn Tyr Lys Ile Ile
   785                 790                 795                 800
   Thr Met Cys Phe Ser Val Ser Leu Ser Ala Thr Val Ala Leu Gly Cys
                   805                 810                 815
   Met Phe Val Pro Lys Val Tyr Ile Ile Leu Ala Lys Pro Glu Arg Asn
               820                 825                 830
   Val Arg Ser Ala Phe Thr Thr Ser Thr Val Val Arg Met His Val Gly
           835                 840                 845
   Asp Gly Lys Ser Ser Ser Ala Ala Ser Arg Ser Ser Ser Leu Val Asn
       850                 855                 860
   Leu Trp Lys Arg Arg Gly Ser Ser Gly Glu Thr Leu Arg Tyr Lys Asp
   865                 870                 875                 880
   Arg Arg Leu Ala Gln His Lys Ser Glu Ile Glu Cys Phe Thr Pro Lys
                   885                 890                 895
   Gly Ser Met Gly Asn Gly Gly Arg Ala Thr Met Ser Ser Ser Asn Gly
               900                 905                 910
   Lys Ser Val Thr Trp Ala Gln Asn Glu Lys Ser Ser Arg Gly Gln His
           915                 920                 925
   Leu Trp Gln Arg Leu Ser Ile His Ile Asn Lys Lys Glu Asn Pro Asn
       930                 935                 940
   Gln Thr Ala Val Ile Lys Pro Phe Pro Lys Ser Thr Glu Ser Arg Gly
   945                 950                 955                 960
   Leu Gly Ala Gly Ala Gly Ala Gly Gly Ser Ala Gly Gly Val Gly Ala
                   965                 970                 975
   Thr Gly Gly Ala Gly Cys Ala Gly Ala Gly Pro Gly Gly Pro Glu Ser
               980                 985                 990
   Pro Asp Ala Gly Pro Lys Ala Leu  Tyr Asp Val Ala Glu  Ala Glu Glu
           995                 1000                 1005
   His Phe  Pro Ala Pro Ala Arg  Pro Arg Ser Pro Ser  Pro Ile Ser
       1010                 1015                 1020
   Thr Leu  Ser His Arg Ala Gly  Ser Ala Ser Arg Thr  Asp Asp Asp
           1025                1030                1035
   Val Pro  Ser Leu His Ser Glu  Pro Val Ala Arg Ser  Ser Ser Ser
       1040                1045                1050
   Gln Gly  Ser Leu Met Glu Gln  Ile Ser Ser Val Val  Thr Arg Phe
       1055                1060                1065
   Thr Ala  Asn Ile Ser Glu Leu  Asn Ser Met Met Leu  Ser Thr Ala
       1070                1075                1080
   Ala Pro  Ser Pro Gly Val Gly  Ala Pro Leu Cys Ser  Ser Tyr Leu
       1085                1090                1095
   Ile Pro  Lys Glu Ile Gln Leu  Pro Thr Thr Met Thr  Thr Phe Ala
       1100                1105                1110
   Glu Ile  Gln Pro Leu Pro Ala  Ile Glu Val Thr Gly  Gly Ala Gln
       1115                1120                1125
   Pro Ala  Ala Gly Ala Gln Ala  Ala Gly Asp Ala Ala  Arg Glu Ser
       1130                1135                1140
   Pro Ala  Ala Gly Pro Glu Ala  Ala Ala Ala Lys Pro  Asp Leu Glu
       1145                1150                1155
   Glu Leu  Val Ala Leu Thr Pro  Pro Ser Pro Phe Arg  Asp Ser Val
       1160                1165                1170
   Asp Ser  Gly Ser Thr Thr Pro  Asn Ser Pro Val Ser  Glu Ser Ala
       1175                1180                1185
   Leu Cys  Ile Pro Ser Ser Pro  Lys Tyr Asp Thr Leu  Ile Ile Arg
       1190                1195                1200
   Asp Tyr  Thr Gln Ser Ser Ser  Ser Leu
       1205                1210
<210> 17
   <211> 381
   <212> PRT
   <213> Artificial Sequence
<220>
   <223> Synthetic
<400> 17
   Met Gly Pro Ile Gly Ala Glu Ala Asp Glu Asn Gln Thr Val Glu Glu
   1               5                   10                  15
   Met Lys Val Glu Gln Tyr Gly Pro Gln Thr Thr Pro Arg Gly Glu Leu
               20                  25                  30
   Val Pro Asp Pro Glu Pro Glu Leu Ile Asp Ser Thr Lys Leu Ile Glu
           35                  40                  45
   Val Gln Val Val Leu Ile Leu Ala Tyr Cys Ser Ile Ile Leu Leu Gly
       50                  55                  60
   Val Ile Gly Asn Ser Leu Val Ile His Val Val Ile Lys Phe Lys Ser
   65                  70                  75                  80
   Met Arg Thr Val Thr Asn Phe Phe Ile Ala Asn Leu Ala Val Ala Asp
                   85                  90                  95
   Leu Leu Val Asn Thr Leu Cys Leu Pro Phe Thr Leu Thr Tyr Thr Leu
               100                 105                 110
   Met Gly Glu Trp Lys Met Gly Pro Val Leu Cys His Leu Val Pro Tyr
           115                 120                 125
   Ala Gln Gly Leu Ala Val Gln Val Ser Thr Ile Thr Leu Thr Val Ile
       130                 135                 140
   Ala Leu Asp Arg His Arg Cys Ile Val Tyr His Leu Glu Ser Lys Ile
   145                 150                 155                 160
   Ser Lys Arg Ile Ser Phe Leu Ile Ile Gly Leu Ala Trp Gly Ile Ser
                   165                 170                 175
   Ala Leu Leu Ala Ser Pro Leu Ala Ile Phe Arg Glu Tyr Ser Leu Ile
               180                 185                 190
   Glu Ile Ile Pro Asp Phe Glu Ile Val Ala Cys Thr Glu Lys Trp Pro
           195                 200                 205
   Gly Glu Glu Lys Ser Ile Tyr Gly Thr Val Tyr Ser Leu Ser Ser Leu
       210                 215                 220
   Leu Ile Leu Tyr Val Leu Pro Leu Gly Ile Ile Ser Phe Ser Tyr Thr
   225                 230                 235                 240
   Arg Ile Trp Ser Lys Leu Lys Asn His Val Ser Pro Gly Ala Ala Asn
                   245                 250                 255
   Asp His Tyr His Gln Arg Arg Gln Lys Thr Thr Lys Met Leu Val Cys
                   260                 265                 270
   Val Val Val Val Phe Ala Val Ser Trp Leu Pro Leu His Ala Phe Gln
           275                 280                 285
   Leu Ala Val Asp Ile Asp Ser Gln Val Leu Asp Leu Lys Glu Tyr Lys
       290                 295                 300
   Leu Ile Phe Thr Val Phe His Ile Ile Ala Met Cys Ser Thr Phe Ala
   305                 310                 315                 320
   Asn Pro Leu Leu Tyr Gly Trp Met Asn Ser Asn Tyr Arg Lys Ala Phe
                   325                 330                 335
   Leu Ser Ala Phe Arg Cys Glu Gln Arg Leu Asp Ala Ile His Ser Glu
               340                 345                 350
   Val Ser Val Thr Phe Lys Ala Lys Lys Asn Leu Glu Val Arg Lys Asn
           355                 360                 365
   Ser Gly Pro Asn Asp Ser Phe Thr Glu Ala Thr Asn Val
       370                 375                 380
<210> 18
   <211> 463
   <212> PRT
   <213> Artificial Sequence
<220>
   <223> Synthetic
<400> 18
   Met Ala Gly Ala Pro Gly Pro Leu Arg Leu Ala Leu Leu Leu Leu Gly
   1               5                   10                  15
   Met Val Gly Arg Ala Gly Pro Arg Pro Gln Gly Ala Thr Val Ser Leu
               20                  25                  30
   Trp Glu Thr Val Gln Lys Trp Arg Glu Tyr Arg Arg Gln Cys Gln Arg
           35                  40                  45
   Ser Leu Thr Glu Asp Pro Pro Pro Ala Thr Asp Leu Phe Cys Asn Arg
       50                  55                  60
   Thr Phe Asp Glu Tyr Ala Cys Trp Pro Asp Gly Glu Pro Gly Ser Phe
   65                  70                  75                  80
   Val Asn Val Ser Cys Pro Trp Tyr Leu Pro Trp Ala Ser Ser Val Pro
                   85                  90                  95
   Gln Gly His Val Tyr Arg Phe Cys Thr Ala Glu Gly Leu Trp Leu Gln
               100                 105                 110
   Lys Asp Asn Ser Ser Leu Pro Trp Arg Asp Leu Ser Glu Cys Glu Glu
           115                 120                 125
   Ser Lys Arg Gly Glu Arg Ser Ser Pro Glu Glu Gln Leu Leu Phe Leu
       130                 135                 140
   Tyr Ile Ile Tyr Thr Val Gly Tyr Ala Leu Ser Phe Ser Ala Leu Val
   145                 150                 155                 160
   Ile Ala Ser Ala Ile Leu Leu Gly Phe Arg His Leu His Cys Thr Arg
                   165                 170                 175
   Asn Tyr Ile His Leu Asn Leu Phe Ala Ser Phe Ile Leu Arg Ala Leu
               180                 185                 190
   Ser Val Phe Ile Lys Asp Ala Ala Leu Lys Trp Met Tyr Ser Thr Ala
           195                 200                 205
   Ala Gln Gln His Gln Trp Asp Gly Leu Leu Ser Tyr Gln Asp Ser Leu
       210                 215                 220
   Ser Cys Arg Leu Val Phe Leu Leu Met Gln Tyr Cys Val Ala Ala Asn
   225                 230                 235                 240
   Tyr Tyr Trp Leu Leu Val Glu Gly Val Tyr Leu Tyr Thr Leu Leu Ala
                   245                 250                 255
   Phe Ser Val Leu Ser Glu Gln Trp Ile Phe Arg Leu Tyr Val Ser Ile
               260                 265                 270
   Gly Trp Gly Val Pro Leu Leu Phe Val Val Pro Trp Gly Ile Val Lys
           275                 280                 285
   Tyr Leu Tyr Glu Asp Glu Gly Cys Trp Thr Arg Asn Ser Asn Met Asn
       290                 295                 300
   Tyr Trp Leu Ile Ile Arg Leu Pro Ile Leu Phe Ala Ile Gly Val Asn
   305                 310                 315                 320
   Phe Leu Ile Phe Val Arg Val Ile Cys Ile Val Val Ser Lys Leu Lys
                   325                 330                 335
   Ala Asn Leu Met Cys Lys Thr Asp Ile Lys Cys Arg Leu Ala Lys Ser
                   340                 345                 350
   Thr Leu Thr Leu Ile Pro Leu Leu Gly Thr His Glu Val Ile Phe Ala
           355                 360                 365
   Phe Val Met Asp Glu His Ala Arg Gly Thr Leu Arg Phe Ile Lys Leu
       370                 375                 380
   Phe Thr Glu Leu Ser Phe Thr Ser Phe Gln Gly Leu Met Val Ala Ile
   385                 390                 395                 400
   Leu Tyr Cys Phe Val Asn Asn Glu Val Gln Leu Glu Phe Arg Lys Ser
                   405                 410                 415
   Trp Glu Arg Trp Arg Leu Glu His Leu His Ile Gln Arg Asp Ser Ser
               420                 425                 430
   Met Lys Pro Leu Lys Cys Pro Thr Ser Ser Leu Ser Ser Gly Ala Thr
           435                 440                 445
   Ala Gly Ser Ser Met Tyr Thr Ala Thr Cys Gln Ala Ser Cys Ser
       450                 455                 460
<210> 19
   <211> 222
   <212> PRT
   <213> Artificial Sequence
<220>
   <223> Synthetic
<400> 19
   Met Leu Trp Leu Leu Phe Phe Leu Val Thr Ala Ile His Ala Glu Leu
   1               5                   10                  15
   Cys Gln Pro Gly Ala Glu Asn Ala Phe Lys Val Arg Leu Ser Ile Arg
               20                  25                  30
   Thr Ala Leu Gly Asp Lys Ala Tyr Ala Trp Asp Thr Asn Glu Glu Tyr
           35                  40                  45
   Leu Phe Lys Ala Met Val Ala Phe Ser Met Arg Lys Val Pro Asn Arg
       50                  55                  60
   Glu Ala Thr Glu Ile Ser His Val Leu Leu Cys Asn Val Thr Gln Arg
   65                  70                  75                  80
   Val Ser Phe Trp Phe Val Val Thr Asp Pro Ser Lys Asn His Thr Leu
                   85                  90                  95
   Pro Ala Val Glu Val Gln Ser Ala Ile Arg Met Asn Lys Asn Arg Ile
               100                 105                 110
   Asn Asn Ala Phe Phe Leu Asn Asp Gln Thr Leu Glu Phe Leu Lys Ile
           115                 120                 125
   Pro Ser Thr Leu Ala Pro Pro Met Asp Pro Ser Val Pro Ile Trp Ile
       130                 135                 140
   Ile Ile Phe Gly Val Ile Phe Cys Ile Ile Ile Val Ala Ile Ala Leu
   145                 150                 155                 160
   Leu Ile Leu Ser Gly Ile Trp Gln Arg Arg Arg Lys Asn Lys Glu Pro
                   165                 170                 175
   Ser Glu Val Asp Asp Ala Glu Asp Lys Cys Glu Asn Met Ile Thr Ile
               180                 185                 190
   Glu Asn Gly Ile Pro Ser Asp Pro Leu Asp Met Lys Gly Gly His Ile
           195                 200                 205
   Asn Asp Ala Phe Met Thr Glu Asp Glu Arg Leu Thr Pro Leu
       210                 215                 220
<210> 20
   <211> 6
   <212> PRT
   <213> Artificial Sequence
<220>
   <223> Synthetic
<400> 20
   Arg Arg Ala Ser Ala Pro
   1               5
<210> 21
   <211> 7
   <212> PRT
   <213> Artificial Sequence
<220>
   <223> Synthetic
<400> 21
   Leu Arg Arg Ala Ser Ala Pro
   1               5
<210> 22
   <211> 8
   <212> PRT
   <213> Artificial Sequence
<220>
   <223> Synthetic
<400> 22
   Trp Leu Arg Arg Ala Ser Ala Pro
   1               5
<210> 23
   <211> 6
   <212> PRT
   <213> Artificial Sequence
<220>
   <223> Synthetic
<400> 23
   Arg Arg Ala Thr Ala Pro
   1               5
<210> 24
   <211> 7
   <212> PRT
   <213> Artificial Sequence
<220>
   <223> Synthetic
<400> 24
   Leu Arg Arg Ala Thr Ala Pro
   1               5
<210> 25
   <211> 8
   <212> PRT
   <213> Artificial Sequence
<220>
   <223> Synthetic
<400> 25
   Trp Leu Arg Arg Ala Thr Ala Pro
   1               5
<210> 26
   <211> 6
   <212> PRT
   <213> Artificial Sequence
<220>
   <223> Synthetic
<400> 26
   Arg Arg Ala Tyr Ala Pro
   1               5
<210> 27
   <211> 7
   <212> PRT
   <213> Artificial Sequence
<220>
   <223> Synthetic
<400> 27
   Leu Arg Arg Ala Tyr Ala Pro
   1               5
<210> 28
   <211> 8
   <212> PRT
   <213> Artificial Sequence
<220>
   <223> Synthetic
<400> 28
   Trp Leu Arg Arg Ala Tyr Ala Pro
   1               5
<210> 29
   <211> 6
   <212> PRT
   <213> Artificial Sequence
<220>
   <223> Synthetic
<400> 29
   Arg Arg Ala Asp Ala Pro
   1 5
<210> 30
   <211> 7
   <212> PRT
   <213> Artificial Sequence
<220>
   <223> Synthetic
<400> 30
   Leu Arg Arg Ala Asp Ala Pro
   1 5
<210> 31
   <211> 8
   <212> PRT
   <213> Artificial Sequence
<220>
   <223> Synthetic
<400> 31
   Trp Leu Arg Arg Ala Asp Ala Pro
   1 5
<210> 32
   <211> 6
   <212> PRT
   <213> Artificial Sequence
<220>
   <223> Synthetic
<400> 32
   Arg Arg Ala Glu Ala Pro
   1 5
<210> 33
   <211> 7
   <212> PRT
   <213> Artificial Sequence
<220>
   <223> Synthetic
<400> 33
   Leu Arg Arg Ala Glu Ala Pro
   1 5
<210> 34
   <211> 8
   <212> PRT
   <213> Artificial Sequence
<220>
   <223> Synthetic
<400> 34
   Trp Leu Arg Arg Ala Glu Ala Pro
   1               5
<210> 35
   <211> 12
   <212> PRT
   <213> Artificial Sequence
<220>
   <223> Synthetic
<400> 35
   Arg Arg Ala Ser Ala Pro Arg Arg Ala Ser Ala Pro
   1               5                   10
<210> 36
   <211> 14
   <212> PRT
   <213> Artificial Sequence
<220>
   <223> Synthetic
<400> 36
   Leu Arg Arg Ala Ser Ala Pro Leu Arg Arg Ala Ser Ala Pro
   1               5                   10
<210> 37
   <211> 16
   <212> PRT
   <213> Artificial Sequence
<220>
   <223> Synthetic
<400> 37
   Trp Leu Arg Arg Ala Ser Ala Pro Trp Leu Arg Arg Ala Ser Ala Pro
   1               5                   10                  15
<210> 38
   <211> 12
   <212> PRT
   <213> Artificial Sequence
<220>
   <223> Synthetic
<400> 38
   Arg Arg Ala Thr Ala Pro Arg Arg Ala Thr Ala Pro
   1               5                   10
<210> 39
   <211> 14
   <212> PRT
   <213> Artificial Sequence
<220>
   <223> Synthetic
<400> 39
   Leu Arg Arg Ala Thr Ala Pro Leu Arg Arg Ala Thr Ala Pro
   1               5                   10
<210> 40
   <211> 16
   <212> PRT
   <213> Artificial Sequence
<220>
   <223> Synthetic
<400> 40
   Trp Leu Arg Arg Ala Thr Ala Pro Trp Leu Arg Arg Ala Thr Ala Pro
   1               5                   10                  15
<210> 41
   <211> 12
   <212> PRT
   <213> Artificial Sequence
<220>
   <223> Synthetic
<400> 41
   Arg Arg Ala Tyr Ala Pro Arg Arg Ala Tyr Ala Pro
   1               5                   10
<210> 42
   <211> 14
   <212> PRT
   <213> Artificial Sequence
<220>
   <223> Synthetic
<400> 42
   Leu Arg Arg Ala Tyr Ala Pro Leu Arg Arg Ala Tyr Ala Pro
   1               5                   10
<210> 43
   <211> 16
   <212> PRT
   <213> Artificial Sequence
<220>
   <223> Synthetic
<400> 43
   Trp Leu Arg Arg Ala Tyr Ala Pro Trp Leu Arg Arg Ala Tyr Ala Pro
   1               5                   10                  15
<210> 44
   <211> 12
   <212> PRT
   <213> Artificial Sequence
<220>
   <223> Synthetic
<400> 44
   Arg Arg Ala Asp Ala Pro Arg Arg Ala Asp Ala Pro
   1               5                   10
<210> 45
   <211> 14
   <212> PRT
   <213> Artificial Sequence
<220>
   <223> Synthetic
<400> 45
   Leu Arg Arg Ala Asp Ala Pro Leu Arg Arg Ala Asp Ala Pro
   1               5                   10
<210> 46
   <211> 16
   <212> PRT
   <213> Artificial Sequence
<220>
   <223> Synthetic
<400> 46
   Trp Leu Arg Arg Ala Asp Ala Pro Trp Leu Arg Arg Ala Asp Ala Pro
   1               5                   10                  15
<210> 47
   <211> 12
   <212> PRT
   <213> Artificial Sequence
<220>
   <223> Synthetic
<400> 47
   Arg Arg Ala Glu Ala Pro Arg Arg Ala Glu Ala Pro
   1               5                   10
<210> 48
   <211> 14
   <212> PRT
   <213> Artificial Sequence
<220>
   <223> Synthetic
<400> 48
   Leu Arg Arg Ala Glu Ala Pro Leu Arg Arg Ala Glu Ala Pro
   1               5                   10
<210> 49
   <211> 16
   <212> PRT
   <213> Artificial Sequence
<220>
   <223> Synthetic
<400> 49
   Trp Leu Arg Arg Ala Glu Ala Pro Trp Leu Arg Arg Ala Glu Ala Pro
   1               5                   10                  15
<210> 50
   <211> 13
   <212> PRT
   <213> Artificial Sequence
<220>
   <223> Synthetic
<400> 50
   Gly Arg Lys Lys Arg Arg Gln Arg Arg Arg Pro Pro Gln
   1               5                   10
<210> 51
   <211> 12
   <212> PRT
   <213> Artificial Sequence
<220>
   <223> Synthetic
<400> 51
   Ala Tyr Ala Arg Ala Ala Ala Arg Gln Ala Arg Ala
   1               5                   10
<210> 52
   <211> 34
   <212> PRT
   <213> Artificial Sequence
<220>
   <223> Synthetic
<400> 52
   Asp Ala Ala Thr Ala Thr Arg Gly Arg Ser Ala Ala Ser Arg Pro Thr
   1 5 10 15
   Glu Arg Pro Arg Ala Pro Ala Arg Ser Ala Ser Arg Pro Arg Arg Pro
   20 25 30
   Val Glu
<210> 53
   <211> 27
   <212> PRT
   <213> Artificial Sequence
<220>
   <223> Synthetic
<400> 53
   Gly Trp Thr Leu Asn Ser Ala Gly Tyr Leu Leu Gly Leu Ile Asn Leu
   1 5 10 15
   Lys Ala Leu Ala Ala Leu Ala Lys Lys Ile Leu
   20 25
<210> 54
   <211> 11
   <212> PRT
   <213> Artificial Sequence
<220>
   <223> Synthetic
<400> 54
   Pro Leu Ser Ser Ile Ser Arg Ile Gly Asp Pro
   1 5 10
<210> 55
   <211> 16
   <212> PRT
   <213> Artificial Sequence
<220>
   <223> Synthetic
<400> 55
   Ala Ala Val Ala Leu Leu Pro Ala Val Leu Leu Ala Leu Leu Ala Pro
   1               5                   10                  15
<210> 56
   <211> 12
   <212> PRT
   <213> Artificial Sequence
<220>
   <223> Synthetic
<400> 56
   Ala Ala Val Leu Leu Pro Val Leu Leu Ala Ala Pro
   1               5                   10
<210> 57
   <211> 15
   <212> PRT
   <213> Artificial Sequence
<220>
   <223> Synthetic
<400> 57
   Val Thr Val Leu Ala Leu Gly Ala Leu Ala Gly Val Gly Val Gly
   1               5                   10                  15
<210> 58
   <211> 21
   <212> PRT
   <213> Artificial Sequence
<220>
   <223> Synthetic
<400> 58
   Gly Ala Leu Phe Leu Gly Trp Leu Gly Ala Ala Gly Ser Thr Met Gly
   1               5                   10                  15
   Ala Trp Ser Gln Pro
               20
<210> 59
   <211> 27
   <212> PRT
   <213> Artificial Sequence
<220>
   <223> Synthetic
<400> 59
   Gly Trp Thr Leu Asn Ser Ala Gly Tyr Leu Leu Gly Leu Ile Asn Leu
   1               5                   10                  15
   Lys Ala Leu Ala Ala Leu Ala Lys Lys Ile Leu
               20                  25
<210> 60
   <211> 18
   <212> PRT
   <213> Artificial Sequence
<220>
   <223> Synthetic
<400> 60
   Lys Leu Ala Leu Lys Leu Ala Leu Lys Ala Leu Lys Ala Ala Leu Lys
   1               5                   10                  15
   Leu Ala
<210> 61
   <211> 21
   <212> PRT
   <213> Artificial Sequence
<220>
   <223> Synthetic
<400> 61
   Lys Glu Thr Trp Trp Glu Thr Trp Trp Thr Glu Trp Ser Gln Pro Lys
   1               5                   10                  15
   Lys Lys Arg Lys Val
               20
<210> 62
   <211> 16
   <212> PRT
   <213> Artificial Sequence
<220>
   <223> Synthetic
<400> 62
   Lys Ala Phe Ala Lys Leu Ala Ala Arg Leu Tyr Arg Lys Ala Gly Cys
   1               5                   10                  15
<210> 63
   <211> 16
   <212> PRT
   <213> Artificial Sequence
<220>
   <223> Synthetic
<400> 63
   Lys Ala Phe Ala Lys Leu Ala Ala Arg Leu Tyr Arg Ala Ala Gly Cys
   1 5 10 15
<210> 64
   <211> 16
   <212> PRT
   <213> Artificial Sequence
<220>
   <223> Synthetic
<400> 64
   Ala Ala Phe Ala Lys Leu Ala Ala Arg Leu Tyr Arg Lys Ala Gly Cys
   1 5 10 15
<210> 65
   <211> 16
   <212> PRT
   <213> Artificial Sequence
<220>
   <223> Synthetic
<400> 65
   Lys Ala Phe Ala Ala Leu Ala Ala Arg Leu Tyr Arg Lys Ala Gly Cys
   1 5 10 15
<210> 66
   <211> 16
   <212> PRT
   <213> Artificial Sequence
<220>
   <223> Synthetic
<400> 66
   Lys Ala Phe Ala Lys Leu Ala Ala Gln Leu Tyr Arg Lys Ala Gly Cys
   1 5 10 15
<210> 67
   <211> 16
   <212> PRT
   <213> Artificial Sequence
<220>
   <223> Synthetic
<400> 67
   Ala Gly Gly Gly Gly Tyr Gly Arg Lys Lys Arg Arg Gln Arg Arg Arg
   1 5 10 15
<210> 68
   <211> 11
   <212> PRT
   <213> Artificial Sequence
<220>
   <223> Synthetic
<400> 68
   Tyr Gly Arg Lys Lys Arg Arg Gln Arg Arg Arg
   1 5 10
<210> 69
   <211> 11
   <212> PRT
   <213> Artificial Sequence
<220>
   <223> Synthetic
<400> 69
   Tyr Ala Arg Ala Ala Ala Arg Gln Ala Arg Ala
   1 5 10
<210> 70
   <211> 20
   <212> DNA
   <213> Artificial Sequence
<220>
   <223> Synthetic
<400> 70
   ggaggggtga agacatgaga 20
<210> 71
   <211> 22
   <212> DNA
   <213> Artificial Sequence
<220>
   <223> Synthetic
<400> 71
   tctgaagtga ttgtccatcc ag 22
<210> 72
   <211> 64
   <212> PRT
   <213> Artificial Sequence
<220>
   <223> Synthetic
<400> 72
   Met Asp Arg Trp Tyr Leu Gly Gly Ser Pro Lys Gly Asp Val Asp Pro
   1               5                   10                  15
   Phe Tyr Tyr Asp Tyr Glu Thr Val Arg Asn Gly Gly Leu Ile Phe Ala
               20                  25                  30
   Gly Leu Ala Phe Ile Val Gly Leu Leu Ile Leu Leu Ser Arg Arg Phe
           35                  40                  45
   Arg Cys Gly Gly Asn Lys Lys Arg Arg Gln Ile Asn Glu Asp Glu Pro
       50                  55                  60
<210> 73
   <211> 37
   <212> PRT
   <213> Artificial Sequence
<220>
   <223> Synthetic
<400> 73
   His Asp Glu Phe Glu Arg His Ala Glu Gly Thr Phe Thr Ser Asp Val
   1               5                   10                  15
   Ser Ser Tyr Leu Glu Gly Gln Ala Ala Lys Glu Phe Ile Ala Trp Leu
               20                  25                  30
   Val Lys Gly Arg Gly
           35
<210> 74
   <211> 33
   <212> PRT
   <213> Artificial Sequence
<220>
   <223> Synthetic
<400> 74
   His Ala Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp
<210> 75
   <211> 99
   <212> PRT
   <213> Artificial Sequence
<220>
   <223> Synthetic
<400> 75
   Met Gln Pro Arg Val Leu Leu Val Val Ala Leu Leu Ala Leu Leu Ala
   1               5                   10                  15
   Ser Ala Arg Ala Ser Glu Ala Glu Asp Ala Ser Leu Leu Ser Phe Met
               20                  25                  30
   Gln Gly Tyr Met Lys His Ala Thr Lys Thr Ala Lys Asp Ala Leu Ser
           35                  40                  45
   Ser Val Gln Glu Ser Gln Val Ala Gln Gln Ala Arg Gly Trp Val Thr
       50                  55                  60
   Asp Gly Phe Ser Ser Leu Lys Asp Tyr Trp Ser Thr Val Lys Asp Lys
   65                  70                  75                  80
   Phe Ser Glu Phe Trp Asp Leu Asp Pro Glu Val Arg Pro Thr Ser Ala
                   85                  90                  95
   Val Ala Ala
<210> 76
   <211> 533
   <212> DNA
   <213> Artificial Sequence
<220>
   <223> Synthetic
<400> 76
   tgctcagttc atccctagag gcagctgctc caggaacaga ggtgccatgc agccccgggt     60
   actccttgtt gttgccctcc tggcgctcct ggcctctgcc cgagcttcag aggccgagga    120
   tgcctccctt ctcagcttca tgcagggtta catgaagcac gccaccaaga ccgccaagga    180
   tgcactgagc agcgtgcagg agtcccaggt ggcccagcag gccaggggct gggtgaccga    240
   tggcttcagt tccctgaaag actactggag caccgttaag gacaagttct ctgagttctg    300
   ggatttggac cctgaggtca gaccaacttc agccgtggct gcctgagacc tcaatacccc   360
   aagtccacct gcctatccat cctgcgagct ccttgggtcc tgcaatctcc agggctgccc    420
   ctgtaggttg cttaaaaggg acagtattct cagtgctctc ctaccccacc tcatgcctgg    480
   cccccctcca ggcatgctgg cctcccaata aagctggaca agaagctgct atg           533